# EUROPEAN PATENT APPLICATION

(11) **EP 4 361 633 A1**
(43) Date of publication of application: **01.05.2024**
(21) Application number: 22383031.6
(22) Date of filing: 25.10.2022
(51) Int. Cl.: G01N 33/574, A61P 35/00

(54) **METHOD FOR PREDICTING RESPONSE TO A CANCER TREATMENT**

(71) Applicant: Peptomyc, S.L., 08035 Barcelona (ES)
(72) Inventor: CASACUBERTA SERRA, Sílvia, E-08035 Barcelona (ES); BEAULIEU, Marie-Eve, E-08035 Barcelona (ES); SOUCEK, Laura, E-08035 Barcelona (ES)
(74) Representative: ABG Intellectual Property Law, S.L.

(57) **Abstract**

The invention relates to a method for predicting the response to an anti-cancer treatment of patients suffering from cancer, said method being based on the detection of biomarkers. The invention also relates to methods of treatment and methods for selecting a cancer patient to be treated or methods for selecting a suitable therapy for a cancer patient based on the detection of said biomarkers.

## Description

### FIELD OF THE INVENTION

The present invention relates to the field of cancer and, more particularly, to methods for predicting the response of a subject diagnosed with cancer to an anti-cancer treatment based on the detection of biomarkers in a sample of the subject. It also relates to methods for selecting a customized therapy for the subject or for selecting a subject to be treated with an anti-cancer agent, and to the anti-cancer agents used in the treatments referred in the methods based on the detection of said biomarkers. It further relates to kits and their use in said methods.

### BACKGROUND OF THE INVENTION

Cancer is a leading cause of death worldwide, accounting for nearly 10 million deaths in 2020. It is a large subset of diseases characterized by the uncontrollable growth of abnormal cells.

Myc is tightly regulated in normal cells, where its levels are higher in proliferating and lower in non-proliferating cells. Aberrantly high and/or deregulated Myc activity is causally implicated in most cancers and often associated with aggressive, poorly differentiated, angiogenic and treatment-resistant tumors.

Omomyc is a dominant-negative Myc mutant comprising the b-HLH-LZ domain of Myc and harboring four amino acid substitutions in the leucine zipper of Myc (Soucek, L. et al., 1998, Oncogene 17, 2463-2472; Soucek, L. et al. (2002), Cancer Res 62: 3507-3510). The amino acid substitutions E61T, E68I, R74Q, and R75N confer altered dimerization specificity to the protein, which retains the ability to bind its natural partner Max and to form homodimers with itself as well as heterodimers with wild type c-, N- and L-Myc.

Because of these properties, Omomyc is able to prevent Myc-dependent gene transactivation functions both *in vitro* and *in vivo* by negating the ability of Myc to bind its DNA recognition binding site, the E box. At the same time, Omomyc strongly potentiates Myc-induced apoptosis in a manner dependent on Myc expression level and thereby strengthens Myc transrepression activity. Omomyc thus prevents Myc binding to promoter E-boxes and transactivation of target genes while retaining Miz-1-dependent binding to promoters and transrepression. In the presence of Omomyc, the Myc interactome is channeled to repression and its activity switches from a prooncogenic to a tumor-suppressive one.

In WO 2014/180889 A8 it was demonstrated that Omomyc peptide itself is capable of efficiently transducing across the cellular membrane and translocate to the nucleus, wherein it exerts its tumor-suppressive effect.

In WO 2018/011433 A1 it was demonstrated that a mutant of Omomyc wherein the only cysteine has been replaced by a different amino acid is even more effective than Omomyc in the treatment of cancer.

However, anticancer therapies can present limitations such as undesired side effects and the emergence of innate or acquired resistance. Selecting those patients that will respond better to a therapy results in an increase of the survival rate of cancer patients, avoiding unnecessary therapeutic side effects and delays in the treatment. Those patients whose tumors have high probability of showing a poor response to an anti-cancer treatment may be candidates for alternative treatments.

Therefore, there is a necessity for a personalized approach for a better treatment of the disease and to identify further useful biomarkers for predicting the response of cancer patients to an anti-cancer treatment.

### BRIEF SUMMARY OF THE INVENTION

In a first aspect, the invention relates to an *in vitro* method for predicting the clinical response of a subject suffering from cancer to an anti-cancer treatment selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
said method comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value,
wherein:
- a decreased level of said at least one biomarker with respect to the reference value is indicative of a good clinical response of the subject to the anti-cancer treatment, or
- an equal or an increased level of said at least one biomarker with respect to the reference value is indicative of a poor clinical response of the subject to the anti-cancer treatment.

In a second aspect, the invention relates to an *in vitro* method for selecting a customized therapy for a subject suffering from cancer comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value,
wherein:
- a decreased level of said at least one biomarker with respect to the reference value is indicative that the therapy to be selected comprises an agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b); or, alternatively,
- an equal or an increased level of said at least one biomarker with respect to the reference value is indicative that the therapy to be selected does not comprise an agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b).

In a third aspect, the invention relates to an *in vitro* method for selecting a subject suffering from cancer for a treatment comprising an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
said method comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value, wherein the patient is selected for said treatment if a decreased level of said at least one biomarker with respect to the reference value is detected.

In a fourth aspect, the invention relates to an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
for use in the treatment of cancer in a subject, wherein the subject has been identified as a good responder to said agent by the first method of the invention or wherein the treatment has been selected by the second method of the invention.

In a further aspect, the invention relates to a kit comprising a reagent specific for determining the expression level of at least one biomarker selected from the group consisting of MIP-1β, CD62E, IL-8, GM-CSF and IL-1α.

In yet another aspect, the invention relates to the use of a kit of the invention or of a reagent specific for determining the expression level of at least one biomarker selected from the group consisting of of MIP-1β, CD62E, IL-8, GM-CSF and IL-1α in the first, second and third methods of the invention.

### DESCRIPTION OF THE FIGURES

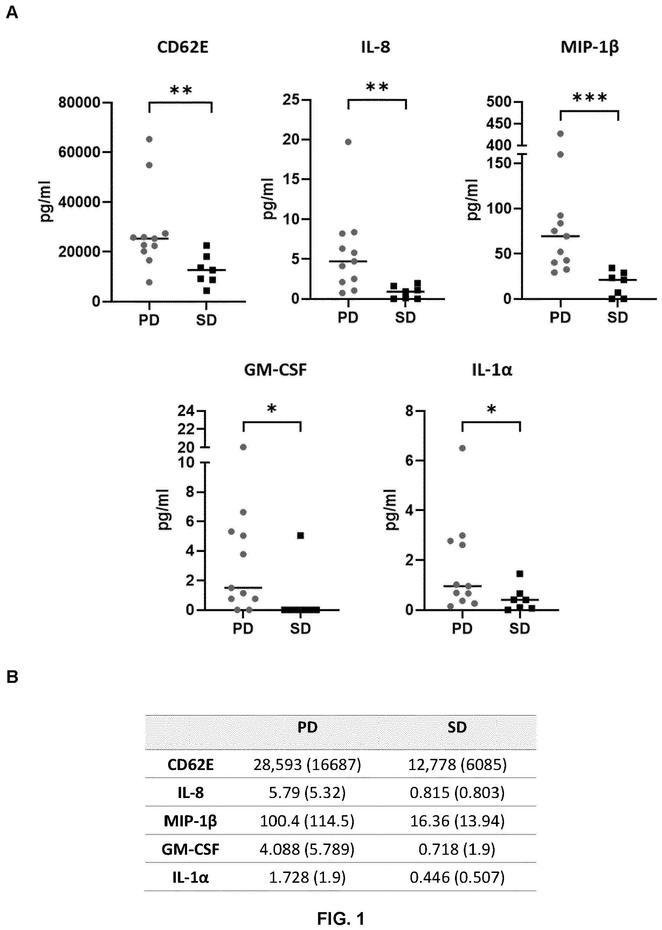

**Figure 1****. Patients that responded to OMO-103 treatment display low levels of CD62E, IL-8, MIP-1β, GM-CSF and IL-1α at baseline.** The levels of the different cytokines and chemokines were measured in the patient serum samples taken at pretreatment using the Luminex technique. **A)** Patients that responded to the treatment and presented Stable Disease (SD) at cycle 3 showed significantly lower levels of CD62E, IL-8, MIP-1β, GM-CSF and IL-1α compared to those showing Progressive Disease (PD). **B)** Levels in pg/ml of the different cytokines and chemokines measured at pretreatment. Mean and Standard deviation is shown.

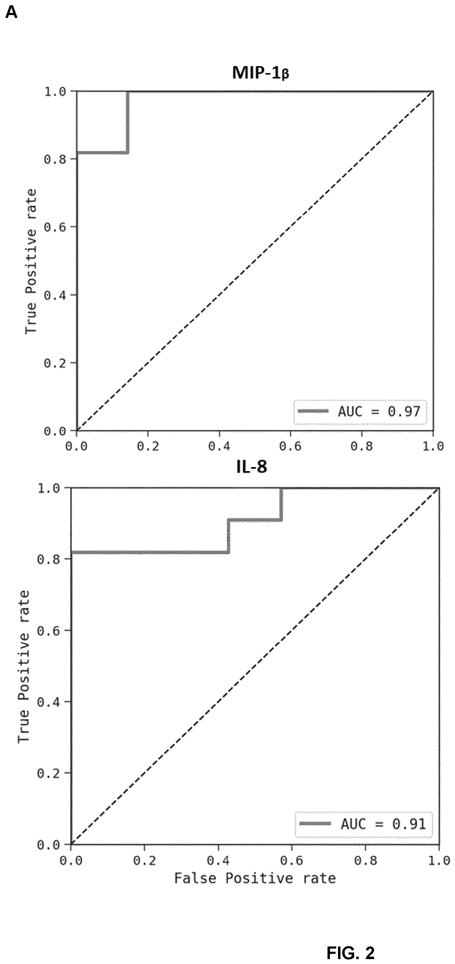

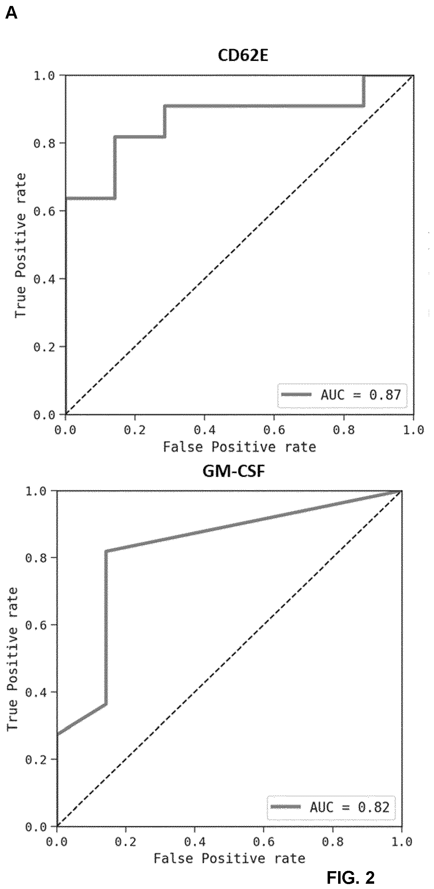

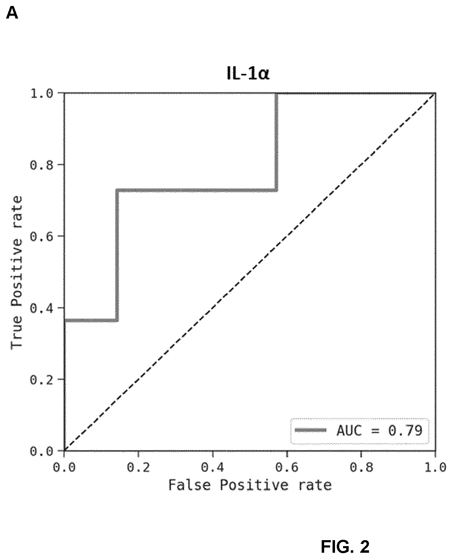

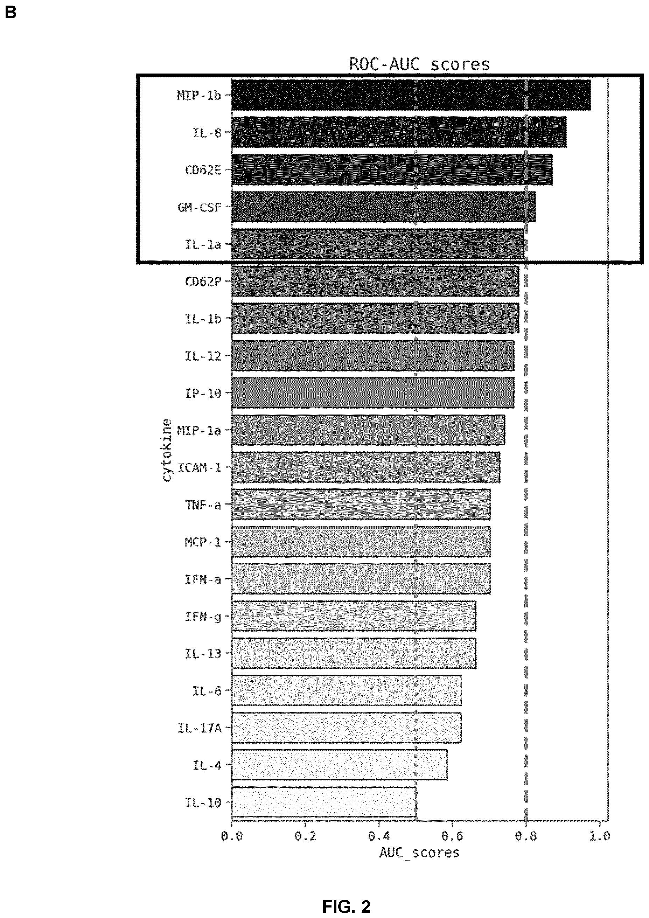

**Figure 2****. CD62E, IL-8, MIP-1β, GM-CSF and IL-1α are excellent predictors of response to OMO-103.** Receiver Operating Characteristic (ROC) curve analysis for individual cytokines to predicting response to OMO-103. **A)** Individual ROC curve graphs. **B)** ROC-AUC scores for each individual cytokine/chemokine. The dotted line indicates an AUC score of 0.5, and the dashed line indicates an AUC score of 0.8, a threshold that indicates an excellent predictive power.

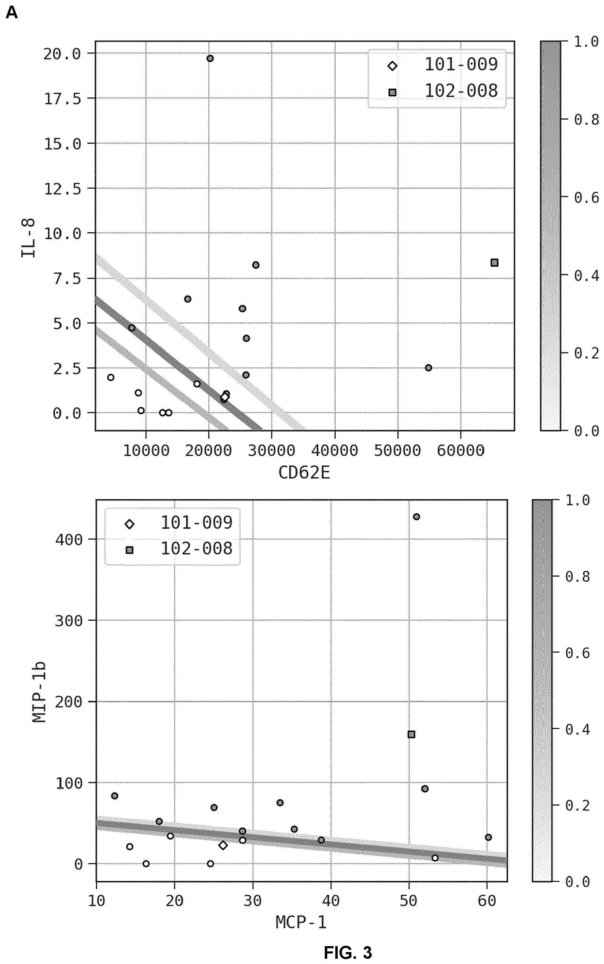

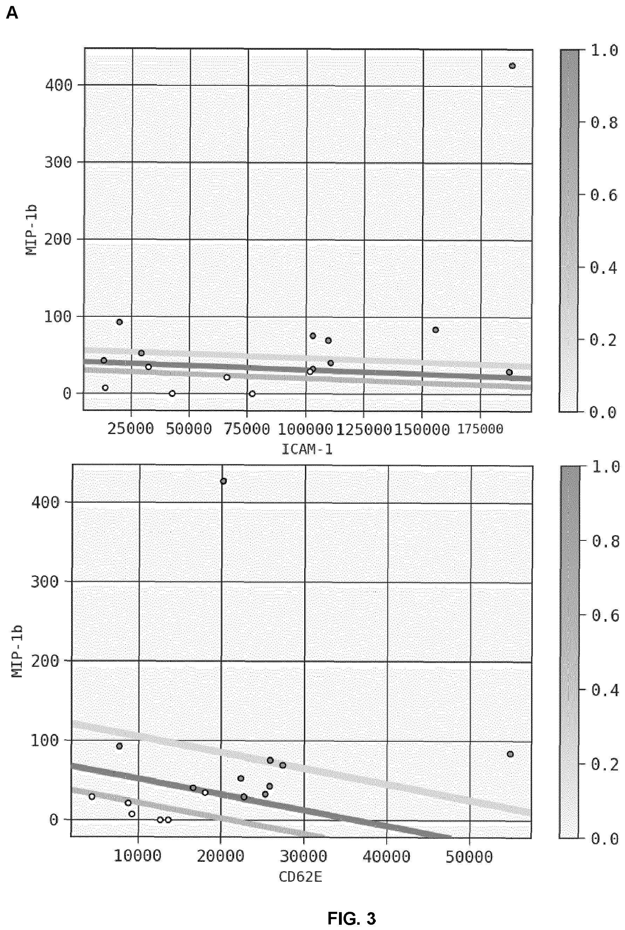

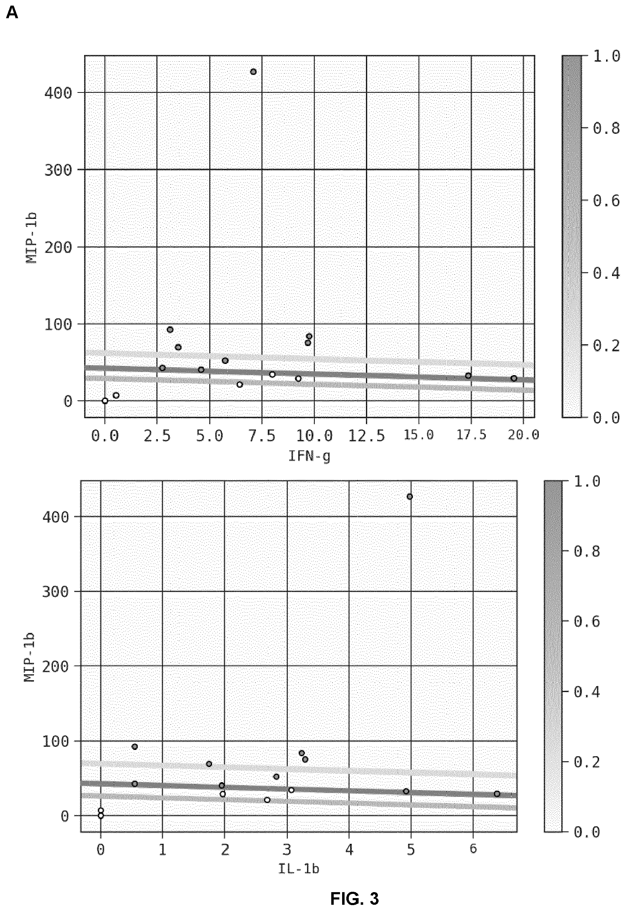

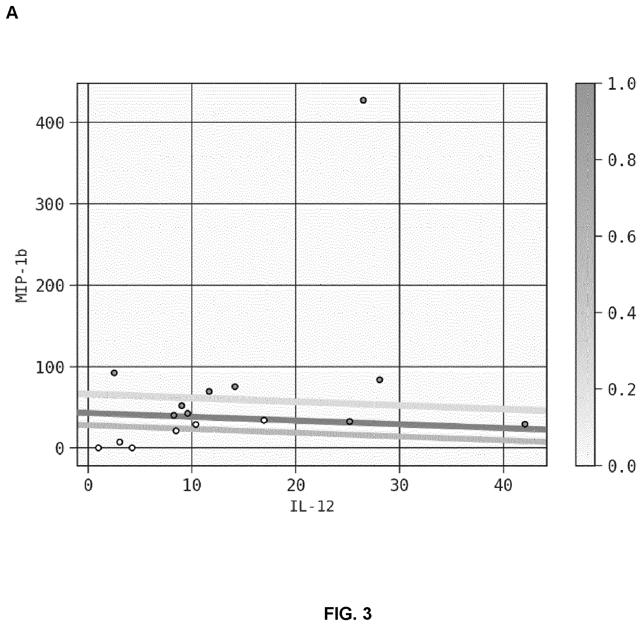

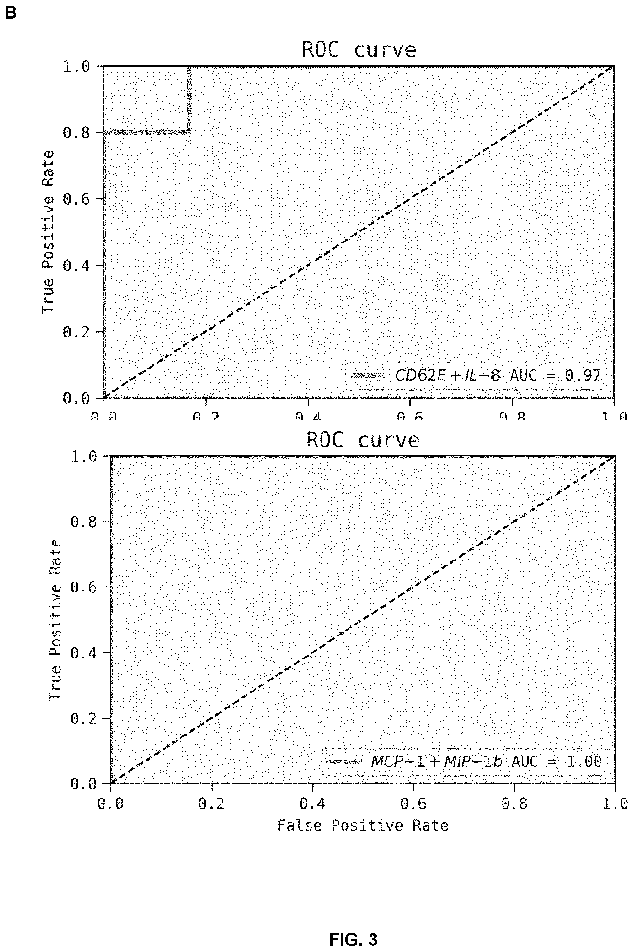

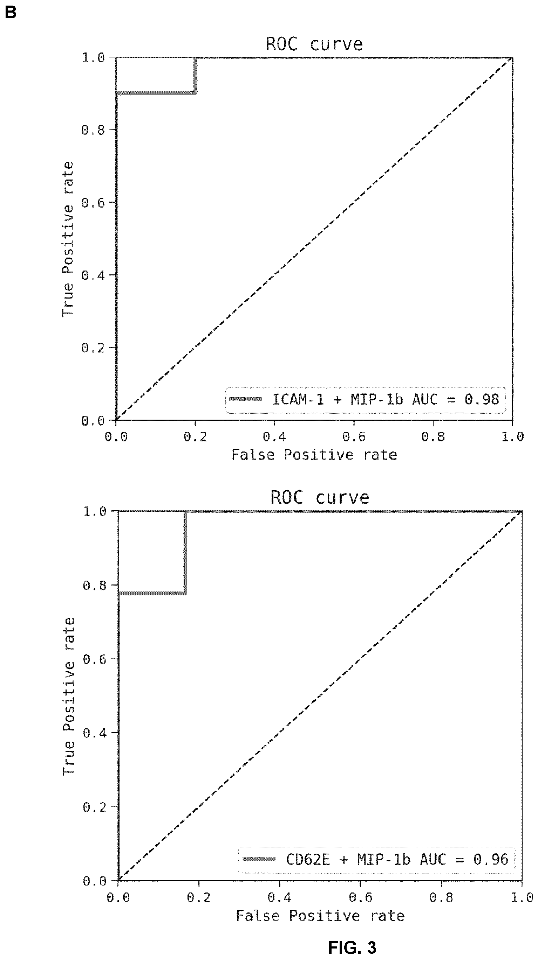

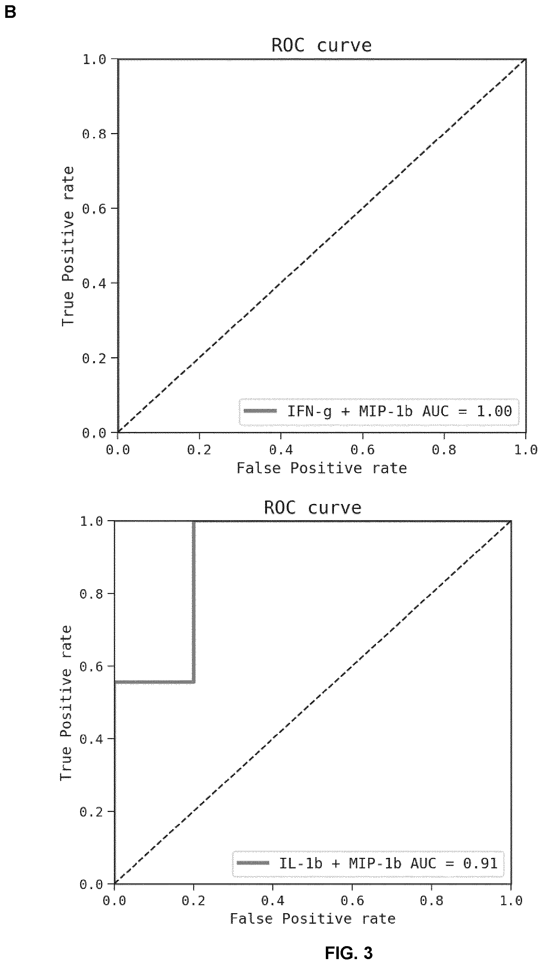

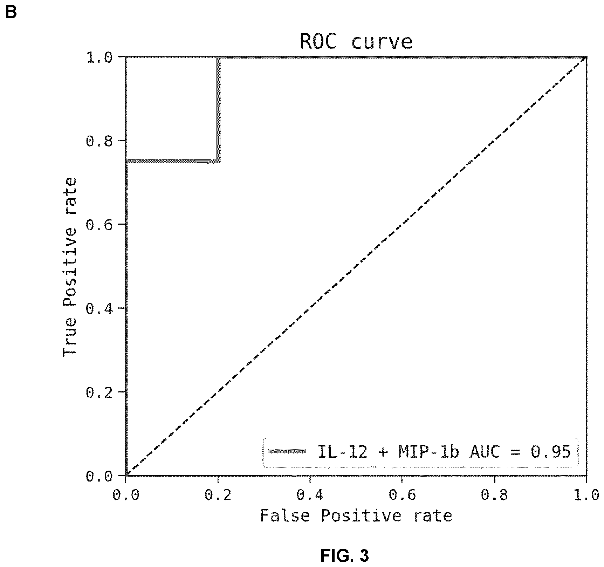

**Figure 3****. Several combinations of two cytokines (CD62E + IL-8, MCP-1 + MIP-1β, MIP-1β + ICAM-1, MIP-1β + CD62E, MIP-1β + IFN-gamma, MIP-1β + IL-1β and MIP-1β + IL-12) are outstanding predictors of response to OMO-103 and can be used to stratify SD and PD patients at pretreatment.** Cytokine/chemokine combination models were generated using the QLattice technology. **A)** Plots of CD62E + IL-8, MCP-1 + MIP-1β, MIP-1β + ICAM-1, MIP-1β + CD62E, MIP-1β + IFN-gamma, MIP-1β + IL-1β and MIP-1β + IL-12 combination models and how they stratify the patients are shown. Stable Disease (SD) is indicated by empty dots/zero and Progressive Disease (PD) is indicated by grey/one. The grey lines correspond to confidence bands (CI), with the dark one indicating the mean, the mid-grey the 5% CI and the clear grey the 95% CI. **B)** Receiver Operating Characteristic (ROC) curve analysis of the seven combination models.

### DETAILED DESCRIPTION OF THE INVENTION

The inventors of the present invention have discovered that, surprisingly, the levels of biomarkers MIP-1β, CD62E, IL-8, GM-CSF and IL-1α are lower in a pretreatment serum sample of subjects suffering from cancer that respond better to a therapy with a polypeptide comprising Omomyc.

Therefore, these biomarkers have potential value to predict a good response to a treatment based in Omomyc or a functionally equivalent variant thereof in cancer patients. Based on these findings, the inventors have developed the methods of the present invention in their different embodiments that will be described now in detail.

The results provided in the examples of the present invention clearly show a significant association of the low level of MIP-1β, CD62E, IL-8, GM-CSF and IL-1α with a good response to a treatment with OMO-103. Thus, these results suggest that subjects suffering from cancer having low levels of these biomarkers in a pretreatment sample are candidates for a treatment based on Omomyc or a functionally equivalent variant thereof.

### PREDICTIVE METHODS OF THE INVENTION

In a first aspect, the invention relates to an *in vitro* method for predicting the clinical response of a subject suffering from cancer to an anti-cancer treatment selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
said method comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value, wherein:
   - a decreased level of said at least one biomarker with respect to the reference value is indicative of a good clinical response of the subject to the anti-cancer treatment, or
   - an equal or an increased level of said at least one biomarker with respect to the reference value is indicative of a poor clinical response of the subject to the anti-cancer treatment.

The term *"in vitro",* as used herein, refers to the fact that an experimental protocol or a method is not carried out on the body of a human or animal subject, but on samples isolated from said subject, and already present in a laboratory tool, such as a test tube, dish or plate.

The term "predicting", as used herein, refers to the determination of the likelihood that the subject suffering from cancer will respond favorably to a therapy with an anti-cancer treatment. Especially, the term "prediction", as used herein, relates to an individual assessment of the expected response of a subject suffering from cancer if the tumor is treated with an anti-cancer treatment based on Omomyc or a functionally equivalent variant thereof as defined in items (a) to (e) of the first aspect of the invention.

The term "clinical response", as used herein, refers to the response of the subject suffering from cancer to an anti-cancer treatment based on Omomyc or a functionally equivalent variant thereof as defined in items (a) to (e) of the first aspect of the invention. Standard criteria (Eisenhauer, E.A., et al. 2009. New response evaluation criteria in solid tumours: revised RECIST guideline (version 1.1). Eur J Cancer 45(2): 228-247) that can be used herewith to evaluate the response to an anti-cancer therapy include response, stabilization and progression. The term "RECIST, as used herein, refers to the "Response Evaluation Criteria in Solid Tumors" and it is a standard way to measure how well a cancer patient responds to treatment. It is based on whether tumors shrink, stay the same, or get bigger. To use RECIST, there must be at least one tumor that can be measured on x-rays, computed tomography (CT) scans, or magnetic resonance imaging (MRI) scans. The types of response a patient can have are a complete response (CR), a partial response (PR), progressive disease (PD), and stable disease (SD). In a preferred embodiment RECIST criteria for tumor response assessment has been used.

In the context of the present invention, subjects achieving complete or partial response and subjects showing a stable disease were considered as "good responders" or having a good clinical response.

A "complete response" (or complete remission) (CR), as used herein, is the disappearance of all detectable malignant disease, i.e., the disappearance of all target lesions, wherein any pathological lymph nodes (whether target or non-target) must have reduction in short axis to <10 mm.

A "partial response" (PR) is defined herein as at least a 30% decrease in the sum of diameters of target lesions, taking as reference the baseline sum diameters.

The term "stabilization", "stabilization of the disease" or "stable disease" (SD), as used herein, is considered when there is neither sufficient shrinkage to qualify for partial response nor sufficient increase to qualify for progressive disease, taking as reference the smallest sum diameters while on study.

In the context of the present invention, subjects showing progressive disease are considered as "poor responders" or having a poor clinical response.

The term "progression" or "progressive disease" (PD), as used herein, is defined as at least a 20% increase in the sum of diameters of target lesions, taking as reference the smallest sum on study (this includes the baseline sum if that is the smallest on study). In addition to the relative increase of 20%, the sum must also demonstrate an absolute increase of at least 5 mm. The appearance of one or more new lesions is also considered progression.

As the person skilled in the art will understand, having a poor response to the anti-cancer treatment does not mean that the subject has no response at all or that the treatment has no effect on the subject. For example, the anti-cancer treatment may reduce the growth of the lesion in a poor responder when compared with a patient that does not undergo any treatment. But the effect achieved in said subject when compared to an untreated patient does not qualify to be considered stabilization under the criteria used in the present invention. For example, a patient treated with an anti-cancer therapy can have an increase of 20% in the sum of diameters of target lesions, but this increase could have been of 30% if the patient is left untreated.

As will be understood by those skilled in the art, such an assessment is usually not intended to be correct for all (i.e. 100 percent) of the subjects to be identified. The term, however, requires that the prediction provides correct results for a statistically significant portion of subjects. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley and Sons, New York 1983. Preferred confidence intervals are at least 50%, at least 60%, at least 70%, at least 80%, at least 90 percent, at least 95 percent, at least 97 percent, at least 98 percent or at least 99 percent. The p- values are, preferably, 0.1, 0.05, 0.01, 0.005, 0.001, 0.0005, 0.0001 or less. More preferably, at least 60 percent, at least 70 percent, at least 80 percent or at least 90 percent of the subjects of a population can be properly identified by the method of the present invention.

Any other parameter which is widely accepted for comparing the efficacy of alternative treatments can be used for determining the response to a treatment and include, without limitation:
- disease-free progression which, as used herein, describes the proportion of subjects in complete remission who have had no recurrence of disease during the time period under study.
- disease-free survival (DFS) which, as used herewith, is understood as the length of time after treatment for a disease during which a subject survives with no sign of the disease.
- objective response which, as used in the present invention, describes the proportion of treated subjects in whom a complete or partial response is observed.
- tumor control which, as used in the present invention, relates to the proportion of treated subjects in whom complete response, partial response, minor response or stable disease ≥ 6 months is observed.
- progression free survival which, as used herein, is defined as the time from start of treatment to the first measurement of cancer growth.
- time to progression (TTP), as used herein, relates to the time after a disease is treated until the disease starts to get worse. The term "progression" has been previously defined.
- six-month progression free survival or "PFS6" rate which, as used herein, relates to the percentage of subjects wherein free of progression in the first six months after the initiation of the therapy.
- median survival which, as used herein, relates to the time at which half of the subjects enrolled in the study are still alive.
- overall survival which, as used herein, refers to the length of time from either the date of diagnosis or the start of treatment for a disease, such as cancer, that patients diagnosed with the disease are still alive.
- recurrence which, as used herein, refers to the appearance of cancer after treatment and after a period of time during which cancer was not detected.
- survival without metastasis, as used herein, refers to the length of time after treatment for a cancer ends that the patient survives without any signs or symptoms of cancer metastasis.
- decrease in circulating tumor cells, as used herein, refers to a decrease in the concentration of circulating tumor cells in the blood or in the lymph of a patient with metastatic cancer. A decrease in circulating tumor cells is associated with efficacy of a therapy against the metastatic cancer.
- response of circulating markers, as used herein, refers to the variation on the concentration in blood or in the lymph of proteins or nucleic acids associated with a specific cancer after treatment against said specific cancer.

The method of the invention is carried out in a subject suffering from cancer, i.e., in a subject that has been previously diagnosed with cancer.

The term "subject" or "patient", as used herein, refers to all animals classified as mammals and includes, but is not restricted to, domestic and farm animals, primates and humans, e.g., human beings, non-human primates, cows, horses, pigs, sheep, goats, dogs, cats or rodents. Preferably, the subject is a male or female human of any age or race. In the context of the present invention, the subject is a subject suffering from cancer or previously diagnosed with cancer. In a preferred embodiment, the subject is a mammal, preferably a human being.

The subjects tested in the first method of the invention have been previously diagnosed with cancer. The term "diagnosed", as used herein, refers to the determination and/or identification of a disease in a subject, i.e., the opinion reached about the disease state of a subject, i.e., the diagnosis opinion. As such, it can also be regarded as an attempt to classify individuals depending on their disease condition. As it will be understood by those skilled in the art, the diagnosis of cancer, although preferred to be, need not be correct for 100% of the subjects to be diagnosed or evaluated. The term, however, requires that a statistically significant portion of subjects identified as such are suffering cancer. Methods to determine if a portion of subjects is statistically significant have been disclosed above in relation to the method of prediction.

The methods of the invention are suitable for any kind of cancer.

The term "cancer" is referred to a group of diseases characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumors are classified as being either benign or malignant: benign tumors are tumors that cannot spread by invasion or metastasis, i.e., they only grow locally; whereas malignant tumors are tumors that are capable of spreading by invasion and metastasis. Biological processes known to be related to cancer include angiogenesis, immune cell infiltration, cell migration and metastasis.

The term "cancer" includes, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), hairy cell leukemia, polycythemia vera, lymphoma (e.g., Hodgkin's disease or non-Hodgkin's disease), CNS lymphoma, AIDS-associated leukemias, Waldenstrom's macroglobulinemia, multiple myeloma, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, mendotheliosarcoma, lymphangiosarcoma, lymphangioendotheliosarcoma, synovioma, mesothelioma, Ewing's tumor, leiomyosarcoma, rhabdomyosarcoma, Kaposi's sarcoma, colon carcinoma, pancreatic cancer, lung cancer, colon cancer, colorectal cancer, bladder cancer, breast cancer, biliary tract cancer, esophageal cancer, stomach/gastric cancer, ovarian cancer including those arising from epithelial cells, stromal cells, germ cells and mesenchymal cells; prostate cancer, oral cancer including squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, teratoma, choriocarcinoma, endometrial/uterine/cervical cancer, seminoma, embryonal carcinoma, Wilm's tumor, cervical cancer, uterine cancer, testicular cancer, lung carcinoma, small cell lung carcinoma, bladder carcinoma, epithelial carcinoma, intraepithelial neoplasms including Bowen's disease and Paget's disease, neuroglioma, glioma, mixed glioma, optic nerve glioma, subependymoma, metastatic brain tumor, pituitary tumors, primitive neuroectodermal (PNET) tumor, juvenile pilocytic astrocytoma (JPA), brain stem glioma, astrocytoma, pineal tumor, rhabdoid tumor, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, and retinoblastoma).

The term "cancer" also includes, without limitation, head and neck cancer, leukemia, cancer of the heart, esophageal cancer, cancer of the small intestine, spleen cancer, kidney cancer, brain cancer, choriocarcinoma, skin cancer, bone cancer, bone marrow, blood, thymus, womb, liver cancer, sarcoma, liposarcoma, fibrosarcoma, Merkel cell carcinoma, Kaposi's sarcoma, testicular cancer including germinal tumors such as seminoma, non-seminoma (teratomas, choriocarcinomas), stromal tumors, and germ cell tumors; thyroid cancer including thyroid adenocarcinoma and medullar carcinoma; renal cancer including adenocarcinoma and Wilms tumor; cholangiocarcinoma, glioblastoma, hematological neoplasms including acute lymphocytic and myelogenous leukemia, T-cell acute lymphoblastic leukemia/lymphoma, hairy cell leukemia, chronic myelogenous leukemia, multiple myeloma, AIDS-associated leukemias and adult T-cell leukemia/lymphoma, intraepithelial neoplasms including Bowen's disease and Paget's disease, lymphomas including Hodgkin's disease and lymphocytic lymphomas, oral cancer including squamous cell carcinoma, adenoma, angiosarcoma, astrocytoma, epithelial carcinoma, germinoma, glioma, hemangioendothelioma, hemangiosarcoma, hematoma, hepatoblastoma, medulloblastoma, melanoma, parotid gland cancer, neuroblastoma, hepatobiliary cancer, suprarenal cancer, osteosarcoma, retinoblastoma, rhabdomyosarcoma, and teratoma. Furthermore, this term includes acrolentiginous melanoma, actinic keratosis adenocarcinoma, adenoid cystic carcinoma, adenomas, adenosarcoma, adenosquamous carcinoma, astrocytic tumors, Bartholin gland carcinoma, basal cell carcinoma, bronchial gland carcinoma, capillary carcinoid, carcinoma, carcinosarcoma, cystadenoma, endodermal sinus tumor, endometrial hyperplasia, endometrial stromal sarcoma, endometrioid adenocarcinoma, ependymal sarcoma, Ewing sarcoma, focal nodular hyperplasia, germ cell tumors, glucagonoma, hemangioblastoma, hemangioendothelioma, hemangioma, hepatic adenoma, hepatic adenomatosis, hepatocellular carcinoma, insulinoma, intraepithelial neoplasia, squamous cell intraepithelial neoplasia, invasive squamous-cell carcinoma, large cell carcinoma, leiomyosarcoma, malignant melanoma, malignant mesothelial tumor, medulobastoma, medulloepithelioma, mucoepidermoid carcinoma, neuroblastoma, neuroepithelial adenocarcinoma, nodular melanoma, papillary serous adenocarcinoma, pituitary tumors, plasmacytoma, pseudosarcoma, pulmonary blastoma, renal cell carcinoma, retinoblastoma, rhabdomyosarcoma, serous carcinoma, microcytic carcinoma, soft tissue carcinoma, somatostatin secreting tumor, squamous carcinoma, squamous cell carcinoma, undifferentiated carcinoma, uveal melanoma, verrucous carcinoma, vipoma, Wilm tumor, intracerebral cancer, rectal cancer, astrocytoma, microcytic cancer and non-microcytic cancer, metastatic melanoma, androgen-independent metastatic prostate cancer, androgen-dependent metastatic prostate cancer.

Cancer includes, in another embodiment, without limitation, mesothelioma, hepatobiliary (hepatic and biliary duct), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, non-Hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

In some embodiments, the cancer is selected from hepatocellular carcinoma, ovarian cancer, ovarian epithelial cancer, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical adenoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from hepatocellular carcinoma (HCC), hepatoblastoma, colon cancer, rectal cancer, ovarian cancer, ovarian epithelial cancer, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical adenoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In a preferred embodiment the cancer is a solid tumor.

Examples of solid tumors are sarcoma, carcinoma or lymphoma. Solid tumors generally comprise an abnormal mass of tissue that typically does not include cysts or liquid areas. In some embodiments, the cancer is selected from renal cell carcinoma, or kidney cancer; hepatocellular carcinoma (HCC) or hepatoblastoma, or liver cancer; melanoma; breast cancer; colorectal carcinoma, or colorectal cancer; colon cancer; rectal cancer; anal cancer; lung cancer, such as non-small cell lung cancer (NSCLC) or small cell lung cancer (SCLC); ovarian cancer, ovarian epithelial cancer, ovarian carcinoma, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical carcinoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma. In an embodiment the solid tumor is selected from the group consisting of non-small cell lung cancer (NSCLC), breast cancer and colorectal cancer. In a particular embodiment of the invention, the solid cancer is selected from the group consisting of pancreatic ductal adenocarcinoma (PDAC), non-small cell lung cancer (NSCLC), colorectal cancer (CRC), salivary gland carcinoma, sarcoma, triple negative breast cancer and pleural mesothelioma. In a particular embodiment of the invention, the solid cancer is selected from the group consisting of pancreatic ductal adenocarcinoma (PDAC), non-small cell lung cancer (NSCLC), colorectal cancer (CRC), salivary gland carcinoma and sarcoma.

In some embodiments, the cancer is selected from hepatocellular carcinoma (HCC), hepatoblastoma, colon cancer, rectal cancer, ovarian cancer, ovarian epithelial cancer, ovarian carcinoma, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical carcinoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, the cancer is hepatocellular carcinoma (HCC). In some embodiments, the cancer is hepatoblastoma. In some embodiments, the cancer is colon cancer. In some embodiments, the cancer is rectal cancer. In some embodiments, the cancer is ovarian cancer, or ovarian carcinoma. In some embodiments, the cancer is ovarian epithelial cancer. In some embodiments, the cancer is fallopian tube cancer. In some embodiments, the cancer is papillary serous cystadenocarcinoma. In some embodiments, the cancer is uterine papillary serous carcinoma (UPSC). In some embodiments, the cancer is hepatocholangiocarcinoma. In some embodiments, the cancer is soft tissue and bone synovial sarcoma. In some embodiments, the cancer is rhabdomyosarcoma. In some embodiments, the cancer is osteosarcoma. In some embodiments, the cancer is anaplastic thyroid cancer. In some embodiments, the cancer is adrenocortical carcinoma. In some embodiments, the cancer is pancreatic cancer, or pancreatic ductal carcinoma. In some embodiments, the cancer is pancreatic adenocarcinoma. In some embodiments, the cancer is glioma. In some embodiments, the cancer is malignant peripheral nerve sheath tumors (MPNST). In some embodiments, the cancer is neurofibromatosis-1 associated MPNST. In some embodiments, the cancer is Waldenstrom's macroglobulinemia. In some embodiments, the cancer is medulloblastoma.

In some embodiments, a cancer is a viral-associated cancer, including human immunodeficiency virus (HIV) associated solid tumors, human papilloma virus (HPV)-16 positive incurable solid tumors, and adult T-cell leukemia, which is caused by human T-cell leukemia virus type I (HTLV-I) and is a highly aggressive form of CD4+ T-cell leukemia characterized by clonal integration of HTLV-I in leukemic cells; as well as virus-associated tumors in gastric cancer, nasopharyngeal carcinoma, cervical cancer, vaginal cancer, vulvar cancer, squamous cell carcinoma of the head and neck, and Merkel cell carcinoma.

Other cancers will be known to one of ordinary skill in the art.

The term cancer includes both primary tumors as well as metastatic tumors. In an embodiment the cancer is a primary tumor. The term "primary tumor", as used herein, refers to a tumor that originated in the location or organ in which it is present and did not metastasize to that location from another location. Therefore, in an embodiment the cancer to be treated is a non-metastatic cancer. In another embodiment the cancer is a metastatic cancer or cancer metastasis. In the context of the present invention, "metastasis" is understood as the propagation of a cancer from the organ where it stated to a different organ.

The anti-cancer treatment of the invention is capable of decreasing cell proliferation irrespective of whether the cancer shows increased expression or activity of the Myc protein. In an embodiment, the cancer to be prevented or treated is Myc-induced cancer. In another embodiment, the cancer to be prevented or treated is not a Myc-induced cancer.

The term "treat" or "treatment" refers to a therapeutic treatment, as well as a prophylactic or prevention method, wherein the goal is to prevent or reduce an unwanted physiological change or disease, such as cancer. Beneficial or desired clinical results include, but not limiting, release of symptoms, reduction of the length of the disease, stabilized pathological state (specifically not deteriorated), retardation in the disease's progression, improve of the pathological state and remission (both partial and total), both detectable and not detectable. "Treatment" can mean also to prolong survival, compared to the expected survival if the treatment is not applied. Those who need the treatment include those who are suffering from cancer.

The authors of the present invention have discovered several biomarkers that are significantly decreased in subjects that are going to respond well to a treatment with OMO-103, which is a polypeptide comprising Omomyc and having SEQ ID NO: 4. All the agents based on Omomyc act over the same target. Therefore, the biomarkers of the invention will be significantly decreased also in good responders to a treatment with any other agent based on Omomyc, either administered as a polypeptide, a conjugate with other chemical moieties, a nucleic acid or vector for gene therapy or in cellular therapy as a cell capable of secreting the polypeptide or conjugate to the medium.

Therefore, in the context of the present invention, the method of the invention allows to predict the clinical response to an anti-cancer treatment selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b).

The term "anti-cancer treatment", in the context of the first method of the invention, refers to any treatment comprising the exposure of the subject being treated with a method used to induce death of cancerous cells selected from items (a) to (e) recited above. Preferably, the anti-cancer treatment is administered intravenously; preferably as 30 minutes intravenous infusion. Preferably, the anti-cancer treatment is administered once a week.

In a preferred embodiment, the anti-cancer treatment comprises a polypeptide comprising the sequence SEQ ID NO: 1 or a functionally equivalent variant thereof, more preferably a polypeptide comprising the sequence SEQ ID NO: 1.

The terms "polypeptide" and "peptide" are used interchangeably herein to refer to polymers of amino acids of any length. The polypeptide of the invention can comprise modified amino acids, and it can be interrupted by non-amino acids. In a preferred embodiment the polypeptide is exclusively formed by amino acids. Preferably the polypeptide of item (a) of the anti-cancer treatment has a length between 80 and 500 amino acids, more preferably between 80 and 300 amino acids, more preferably between 80 and 250 amino acids, more preferably between 80 and 150, even more preferably between 80 and 130 amino acids, preferably between 90 and 130 amino acids, preferably no more than 125 amino acids, more preferably no more than 100 amino acids. In a preferred embodiment, the polypeptide has a length between 90 and 98 amino acids, preferably between 90 and 95 amino acids, more preferably 91 amino acids.

The term "amino acid" refers to naturally occurring and synthetic amino acids, as well as amino acid analogs and amino acid mimetics that function in a manner similar to the naturally occurring amino acids. Furthermore, the term "amino acid" includes both D- and L-amino acids (stereoisomers). Preferably, the amino acids are L-amino acids.

The term "natural amino acids" or "naturally occurring amino acids" comprises the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine.

As used herein, the term "non-natural amino acid" or "synthetic amino acid" refers to a carboxylic acid, or a derivative thereof, substituted at position "a" with an amine group and being structurally related to a natural amino acid. Illustrative non- limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxy lysine, alio hydroxy lysine, 3-hydroxyproline, 4-hydroxyproline, isodesmosine, alloisoleucine, N-methylglycine, N-methylisoleucine, 6-N-methyl-lysine, N-methylvaline, norvaline, norleucine, ornithine, etc.

The polypeptide of the present invention may also comprise non-amino acid moieties, such as for example, hydrophobic moieties (various linear, branched, cyclic, polycyclic or heterocyclic hydrocarbons and hydrocarbon derivatives) attached to the peptides; various protecting groups which are attached to the compound's terminals to decrease degradation. Suitable protecting functional groups are described in Green and Wuts, "Protecting Groups in Organic Synthesis", John Wiley and Sons, Chapters 5 and 7, 1991.

Chemical (non-amino acid) groups present in the polypeptide may be included in order to improve various physiological properties such as decreased degradation or clearance; decreased repulsion by various cellular pumps, improve various modes of administration, increased specificity, increased affinity, increased stability, bioavailability, solubility, decreased toxicity and the like.

"Mimetic" include molecules which mimic the chemical structure of a peptidic structure and retain the functional properties of the peptidic structure. Approaches to designing peptide analogs, derivatives and mimetics are known in the art.

In an embodiment the polypeptide of the invention is a polypeptide consisting of sequence SEQ ID NO: 1 or a polypeptide consisting of a functionally equivalent variant of SEQ ID NO: 1, preferably is a polypeptide consisting of the sequence SEQ ID NO: 1.

The SEQ ID NO: 1 corresponds to

TEENVKRRTHNVLERQRRNELKRSFFALRDQIPELENNEKAPKVVILKKATAYILSVQA
ETQKLISEIDLLRKQNEQLKHKLEQLRNSCA (SEQ ID NO: 1)

The polypeptide of sequence SEQ ID NO: 1 corresponds to the Omomyc protein sequence. The term "Omomyc", as used herein, refers to a polypeptide which consists of a mutated version of the bHLHZip domain of the Myc carrying the E61T, E68I, R74Q and R75N mutations (wherein the numbering of the mutated positions is given with respect to the sequence of Myc region corresponding to amino acids 365-454 of the polypeptide as defined under accession number NP_002458 in the NCBI database, release of March 15, 2015). The sequence of c-Myc provided in the NCBI database under the accession number NP_002458 is shown below (SEQ ID NO: 2), wherein the region from which Omomyc derives is shown underlined:

1 MDFFRVVENQ QPPATMPLNV SFTNRNYDLD YDSVQPYFYC DEEENFYQQQ QQSELQPPAP

61 SEDIWKKFEL LPTPPLSPSR RSGLCSPSYV AVTPFSLRGD NDGGGGSFST ADQLEMVTEL

121 LGGDMVNQSF ICDPDDETFI KNIIIQDCMW SGFSAAAKLV SEKLASYQAA RKDSGSPNPA

181 RGHSVCSTSS LYLQDLSAAA SECIDPSVVF PYPLNDSSSP KSCASQDSSA FSPSSDSLLS

241 STESSPQGSP EPLVLHEETP PTTSSDSEEE QEDEEEIDVV SVEKRQAPGK RSESGSPSAG

301 GHSKPPHSPL VLKRCHVSTH QHNYAAPPST RKDYPAAKRV KLDSVRVLRQ ISNNRKCTSP

361 RSSDTEENVK RRTHNVLERQ RRNELKRSFF ALRDQIPELE NNEKAPKVVI LKKATAYILS

421 VQAEEQKLIS EEDLLRKRRE QLKHKLEQLR NSCA (SEQ ID NO: 2)

Omomyc also contains the M2 domain of c-Myc, having the sequence RQRRNELKRSF (SEQ ID NO: 3) (see Dang and Lee, Mol.Cell. Biol., 1988, 8:4048-4054) (double underlined above), and which corresponds to a nuclear localization signal.

Omomyc is characterized in that it shows increased dimerization capacity with all three oncogenic Myc proteins (c-Myc, N-Myc and L-Myc). Omomyc can derive from the bHLHZip domain of any Myc protein known in the art, provided that the mutations which result in the tumor suppressor effect are preserved. Thus, the Omomyc that can be used in the present invention may derive from any mammal species, including but not being limited to domestic and farm animals (cows, horses, pigs, sheep, goats, dog, cats or rodents), primates and humans. Preferably, the Omomyc protein is derived from human Myc protein (accession number NP_002458, release of March 12, 2019).

The term "Myc", as used, herein, refers to a family of transcription factors which includes c-Myc, N-Myc and L-Myc. Myc protein activates expression of many genes through binding on consensus sequence CACGTG (Enhancer Box sequences or E-boxes and recruiting histone acetyl-transferases or HATs). However, Myc can also act as a transcriptional repressor. By binding the Miz-1 transcription factor and displacing p300 co-activator, it inhibits expression of Miz-1 target genes. Myc also has a direct role in the control of DNA replication.

The Myc b-HLH-LZ or Myc basic region helix-loop-helix leucine zipper domain refers to a region which determines Myc dimerization with Max protein and binding to Myc-target genes. This region corresponds to amino acids 365-454 of human Myc and is characterized by two alpha helices connected by a loop (Nair, S. K., & Burley, S. K., 2003, Cell, 112: 193-205).

In a preferred embodiment, the polypeptide of the invention is a polypeptide that comprises, consists of or consists essentially of the SEQ ID NO: 4 shown below.

MTEENVKRRTHNVLERQRRNELKRSFFALRDQIPELENNEKAPKVVILKKATAYILSVQ
AETQKLISEIDLLRKQNEQLKHKLEQLRNSCA (SEQ ID NO: 4)

In this context, "consisting essentially of" means that the specified molecule would not contain any additional sequences that would alter the activity of SEQ ID NO: 4.

Preferably, the polypeptide consists of SEQ ID NO: 4.

In a preferred embodiment, the anti-cancer treatment comprises the use of a polypeptide consisting of SEQ ID NO: 4

The term "functionally equivalent variant", refers to any polypeptide which results from the insertion or addition of one or more amino acids and/or from the deletion of one or more amino acids and/or from the conservative substitution of one or more amino acids with respect to the polypeptide of SEQ ID NO: 1 and/or which results from the chemical modification of the polypeptide of SEQ ID NO: 1 and which substantially preserves the tumor suppressor activity of the SEQ ID NO: 1. Preferably, the functionally equivalent variant refers to any polypeptide which results from the insertion or addition of one or more amino acids and/or from the deletion of one or more amino acids and/or from the conservative substitution of one or more amino acids with respect to the polypeptide of SEQ ID NO: 1 and which substantially preserves the tumor suppressor activity of SEQ ID NO: 1; more preferably results from the insertion or addition of one or more amino acids with respect to the polypeptide of SEQ ID NO: 1.

The skilled person will understand that the preservation of the tumor suppressor activity requires that the variant can dimerize with Myc and/or its obligate partner p21/p22Max and inhibit Myc activity, that it is capable of translocating across the cell membrane and that it is capable of translocating across the nuclear envelope. In some embodiments, the functionally equivalent variant of the polypeptide of the invention homodimerizes less than Omomyc, or is not forced into homodimers by the formation of disulphide bridge. In particular the disulphide bridge formation in the homodimer form of certain embodiments of the polypeptide of the invention is less than in the polypeptide Omomyc.

"Less homodimerization", as used herein, relates to the lower ability of forming obligate homodimers of the polypeptide of the invention even in reducing conditions. In a preferred embodiment, the ability is at least 5%, at least 10%, at least 15%, at least 20%, at least 25%, at least 30%, at least 35%, at least 40%, at least 45 %, at least 50%, at least 55%, at least 60%, at least 65%, at least 70%, at least 75%, at least 80%, at least 85%, at least 90%, at least 95% less than the ability of forming homodimers of Omomyc.

Reducing conditions, as used herein, relates to the presence of a reducing agent, a compound that donates an electron to another chemical species in a redox chemical reaction. Illustrative, non-limitative examples of reducing agents are DTT (dithiothreitol), b-mercaptoethanol or TCEP (tris(2-carboxyethyl)phosphine). It is possible that the amount of homodimers is the same *in vitro,* and that the difference between the functionally equivalent variant and Omomyc is present only in cells in presence of heterodimerization partners where the absence of the disulfide enables a potentially higher formation of heterodimers.

Several assays may be used to determine the homodimerization of a peptide, by way of illustrative non-limitative example by thermal denaturation monitored by circular dichroism, so dimerization may be detected through folding and thermal stability quantification.

Suitable functionally equivalent variants include polypeptides consisting essentially of the polypeptide of SEQ ID NO: 1. In this context, "consisting essentially of" means that the specified molecule would not contain any additional sequences that would alter the activity of the SEQ ID NO: 1.

In a preferred embodiment, the functionally equivalent variant of SEQ ID NO: 1 is a polypeptide which results from the insertion or addition of one or more amino acids with respect to the polypeptide of SEQ ID NO: 1. In an embodiment, the functionally equivalent variant results from the insertion of less than 10 amino acids, more preferably less than 5 amino acids, more preferably results from the insertion of one amino acid. In a preferred embodiment, results from the insertion of one amino acid that is methionine.

In another embodiment, the functionally equivalent variant of SEQ ID NO: 1 is a polypeptide which results from the deletion of one or more amino acids with respect to the polypeptide of SEQ ID NO: 1. In an embodiment, the functionally equivalent variant results from the deletion of less than 10 amino acids, more preferably less than 5 amino acids, more preferably results from the deletion of one amino acid.

Suitable functional variants of the targeting peptide are those showing a degree of identity with respect to the peptide of SEQ ID NO:1 of about greater than 25% amino acid sequence identity, such as 25%, 30%, 40%, 50%, 60%, 65%, 70%, 75%, 80%, 85%, 90%, 91 %, 92%, 93%, 94%, 95%, 96%, 97%, 98% or 99%. The degree of identity between two polypeptides is determined using computer algorithms and methods that are widely known for the persons skilled in the art. The identity between two amino acid sequences is preferably determined by using the BLASTP algorithm as described previously (BLAST Manual, Altschul, S., et al., NCBI NLM NIH Bethesda, Md. 20894, Altschul, S., et al., J. Mol. Biol. 1990;215: 403-410). In a preferred embodiment, the sequence identity is determined throughout the whole length of the polypeptide of SEQ ID NO: 1 or throughout the whole length of the variant or of both.

The functionally equivalent variants of the polypeptide of the invention may also include post-translational modifications, such as glycosylation, acetylation, isoprenylation, myristoylation, proteolytic processing, etc.

In another embodiment, suitable functional variants of the targeting peptide are those wherein one or more positions within the polypeptide of the invention contain an amino acid which is a conservative substitution of the amino acid present in the protein mentioned above. "Conservative amino acid substitutions" result from replacing one amino acid with another having similar structural and/or chemical properties. For example, the following six groups each contain amino acids that are conservative substitutions for one another: 1) Alanine (A), Serine (S), Threonine (T); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); and 6) Phenylalanine (F), Tyrosine (Y), Tryptophan (W). Selection of such conservative amino acid substitutions is within the skill of one of ordinary skill in the art and is described, for example, by Dordo et al., (J. Mol. Biol, 1999, 217;721-739) and Taylor et al., (J. Theor. Biol., 1986, 119:205-218).

It will be understood that in a preferred embodiment the functionally equivalent variants of Omomyc contain mutations at positions corresponding to the mutations E61T, E68I, R74Q and R75N found in Omomyc derived from human c-Myc. The position wherein said mutations have to occur in the functionally equivalent variant can be determined by a multiple sequence alignment of different Myc sequences and identifed by the alignment of those positions corresponding to positions 61, 68, 74 and 75 within the sequence of Omomyc derived from human c-Myc. In an embodiment, the functionally equivalent variants of Omomyc contain mutations at positions corresponding to the mutations E61T, E68I, R74Q and R75N found in Omomyc derived from human c-Myc.

In another embodiment, the functionally equivalent variants of Omomyc contain mutations at positions corresponding to E61, E68, R74 and R75 within the sequence of Omomyc wherein E61 has been mutated to E61A or E61S; E68 has been mutated to E68L, E68M or E68V; R74 has been mutated to R74N; and R75 has been mutated to R75Q.

A multiple sequence alignment is an extension of pairwise alignment to incorporate more than two sequences at a time. Multiple alignment methods align all of the sequences in a given query set. A preferred multiple sequence alignment program (and its algorithm) is ClustalW, Clusal2W or ClustalW XXL (see Thompson et al. (1994) Nucleic Acids Res 22:4673-4680). Once the sequences of c-Myc from different organisms and of the variant are compared (aligned) as described herein, the skilled artisan can readily identify the positions within each of the sequence corresponding to positions E61T, E68I, R74Q and R75N found in Omomyc and introduce within the Omomyc variant mutations corresponding to the E61T, E68I, R74Q and R75N mutations found in Omomyc derived from human c-Myc.

Suitable assays for determining whether a polypeptide can be considered as a functionally equivalent variant of Omomyc include, without limitation:
- Assays which measure the capacity of the polypeptide to form dimeric complexes with Max and Myc, such as the assays based on the expression of a reporter gene as described in Soucek et al. (Oncogene, 1998, 17: 2463 - 2472) as well as PLA (protein Ligation assay) or co-immunoprecipitation.
- Assays which measure the capacity of the polypeptide to bind to the Myc/Max recognition site within DNA (the CACGTG site), such as the electrophoretic mobility shift assay (EMSA) described in Soucek et al. (supra.)
- Assays which measure the capacity to repress Myc-induced transactivation, such as the assay based on the expression of a reporter gene under the control of the DNA binding sites specific for Myc/Max as described by Soucek et al. (supra.).
- Assays based on the capacity of the polypeptide to inhibit growth of cells expressing the myc oncogene, as described by Soucek et al. (supra.).
- Assays which measure the ability of the polypeptide to enhance myc-induced apoptosis, such as the assays described by Soucek et al. (Oncogene, 1998: 17, 2463 - 2472). Moreover, any assay commonly known in the art for assessing apoptosis in a cell can be used, such as the Hoechst staining, Propidium Iodide (PI) or Annexin V staining, trypan blue, DNA laddering/fragmentation and TUNEL.

In a preferred embodiment, a polypeptide is considered a functionally equivalent variant of Omomyc if it shows an activity in one or more of the above assays which is at least 10%, 20%, 30%, 40%, 50%, 60%, 70%, 80%, 90% or 100% of the native Omomyc.

In a particular embodiment, the functionally equivalent variant of the polypeptide of SEQ ID NO: 1 comprises the polypeptide of SEQ ID NO: 1, wherein the residue X at position 89 of SEQ ID NO: 1 is not a cysteine. Preferably, the residue X at position 89 of SEQ ID NO: 1 is an aliphatic amino acid, or a sulfured amino acid, or a dicarboxylic amino acid or their amides, or an amino acid having two basic groups, or an aromatic amino acid, or a cyclic amino acid, or a hydroxylated amino acid. More preferably is an amino acid selected from serine, threonine and alanine, preferably selected from serine and alanine.

Suitable functionally equivalent variants of SEQ ID NO: 1 having a residue X at position 89 of SEQ ID NO: 1 which is not a cysteine are disclosed in the following table.

| **SEQ ID NO** | **SEQUENCE** |
|---|---|
| SEQ ID NO: 5 | TEENVKRRTHNVLERQRRNELKRSFFALRDQIPELENNEKAPKVVILKKATAYILSV QAETQKLISEIDLLRKQNEQLKHKLEQLRNSXA (wherein X is any aa different from Cys) |
| SEQ ID NO: 6 | MTEENVKRRTHNVLERQRRNELKRSFFALRDQIPELENNEKAPKVVILKKATAYILS VQAETQKLISEIDLLRKQNEQLKHKLEQLRNSXA (wherein X is any aa different from Cys) |
| SEQ ID NO: 7 | TEENVKRRTHNVLERQRRNELKRSFFALRDQIPELENNEKAPKVVILKKATAYILSV QAETQKLISEIDLLRKQNEQLKHKLEQLRNSSA |
| SEQ ID NO: 8 | MTEENVKRRTHNVLERQRRNELKRSFFALRDQIPELENNEKAPKVVILKKATAYILS VQAETQKLISEIDLLRKQNEQLKHKLEQLRNSSA |
| SEQ ID NO: 9 | TEENVKRRTHNVLERQRRNELKRSFFALRDQIPELENNEKAPKVVILKKATAYILSV QAETQKLISEIDLLRKQNEQLKHKLEQLRNSAA |
| SEQ ID NO: 10 | MTEENVKRRTHNVLERQRRNELKRSFFALRDQIPELENNEKAPKVVILKKATAYILS VQAETQKLISEIDLLRKQNEQLKHKLEQLRNSAA |

Thus, in a preferred embodiment, the functionally equivalent variant of the polypeptide of SEQ ID NO: 1 is selected from the group consisting of SEQ ID NO: 4, SEQ ID NO: 5, SEQ ID NO: 6, SEQ ID NO: 7, SEQ ID NO: 8, SEQ ID NO: 9 and SEQ ID NO: 10. Preferably, the functionally equivalent variant is SEQ ID NO: 4.

Additionally, functionally equivalent variants of Omomyc are also capable of transducing cells after the variant is contacted with said cell. It will be understood that functionally equivalent variants of Omomyc contain the protein transducing domain found in native Omomyc or another functional protein transducing domain.

In a preferred embodiment, a polypeptide is considered as a functionally equivalent variant of SEQ ID NO: 1 if it is capable of transducing a target cell at least 10%, 20%, 30%, 40%, 50%, 60%, 70%, 80%, 90% or 100% as efficiently as SEQ ID NO: 1.

Additionally, functionally equivalent variants of SEQ ID NO: 1 are also capable of translocating to the nucleus of the target tumor cell.

In a preferred embodiment, a polypeptide is considered as a functionally equivalent variant of SEQ ID NO: 1 if it is capable of translocating to the nucleus of the target tumor cells at least 10%, 20%, 30%, 40%, 50%, 60%, 70%, 80%, 90% or 100% as efficiently as the SEQ ID NO: 1.

Suitable assays for determining whether a polypeptide is a functionally equivalent variant of SEQ ID NO: 1 in terms of its ability to translocate across the cellular membrane and to the nucleus include double labelling of a cell with a reagent specific for the polypeptide and with a dye which specifically labels the nucleus of the cell (such as DAPI or Hoechst dye). The detection of the polypeptide of the invention can be performed by confocal microscopy or by fluorescence microscopy.

In another preferred embodiment, the anti-cancer treatment is a conjugate comprising a polypeptide comprising the sequence SEQ ID NO: 1 or a functionally equivalent variant thereof and a chemical moiety that facilitates cellular uptake of the polypeptide or of the functionally equivalent variant thereof.

The term "conjugate", as used herein, refers to two or more compounds which are covalently linked together so that the function of each compound is retained in the conjugate.

The term "chemical moiety" refers to any chemical compound containing at least one carbon atom. Examples of chemical moieties include, but are not limited to, any peptide chain enriched in hydrophobic amino acids and hydrophobic chemical moieties.

In preferred embodiments, the conjugate according to the invention comprises at least 1, at least 2, at least 3, at least 4, at least 5, at least 6, at least 7, at least 8, at least 9, at least 10 or more chemical moieties that facilitate cellular uptake of the polypeptide or of the functionally equivalent variant of said polypeptide.

In one embodiment, the chemical moiety that facilitates cellular uptake of the polypeptide is a lipid or a fatty acid.

A fatty acid generally is a molecule comprising a carbon chain with an acidic moiety (e.g., carboxylic acid) at an end of the chain. The carbon chain of a fatty acid may be of any length, however, it is preferred that the length of the carbon chain be of at least 2, 3, 4, 5, 6, 7, 8, 9, 10, 11, 12, 13, 14, 15, 16, 17, 18, 19, 20 or more carbon atoms, and any range derivable therein. In certain embodiments, the length of the carbon chain is from 4 to 18 carbon atoms in the chain portion of the fatty acid. In certain embodiments the fatty acid carbon chain may comprise an odd number of carbon atoms, however, an even number of carbon atoms in the chain may be preferred in certain embodiments. A fatty acid comprising only single bonds in its carbon chain is called saturated, while a fatty acid comprising at least one double bond in its chain is called unsaturated. The fatty acid may be branched, though in preferable embodiments of the present invention, it is unbranched. Specific fatty acids include, but are not limited to, linoleic acid, oleic acid, palmitic acid, linolenic acid, stearic acid, lauric acid, myristic acid, arachidic acid, palmitoleic acid, and arachidonic acid.

In a preferred embodiment, the chemical moiety that facilitates the cellular uptake of the polypeptide comprising the sequence SEQ ID NO: 1 or a functionally equivalent variant thereof, is a cell penetrating peptide sequence, in which case, the conjugate would comprise a fusion protein comprising the polypeptide comprising SEQ ID NO: 1 or the functionally equivalent variant thereof and the cell penetrating peptide sequence.

The term "fusion protein" relates to proteins generated by gene technology which consist of two or more functional domains derived from different proteins. A fusion protein may be obtained by conventional means, e.g., by means of gene expression of the nucleotide sequence encoding for said fusion protein in a suitable cell. It will be understood that the cell penetrating peptide refers to a cell penetrating peptide which is different from the cell penetrating peptide which forms part of the polypeptide comprising SEQ ID NO: 1 or of the functionally equivalent variant of SEQ ID NO: 1.

The term "cell penetrating peptide sequence" is used in the present specification interchangeably with "CPP", "protein transducing domain" or "PTD". It refers to a peptide chain of variable length that directs the transport of a protein inside a cell. The delivering process into cell commonly occurs by endocytosis but the peptide can also be internalized into cell by means of direct membrane translocation. CPPs typically have an amino acid composition that either contains a high relative abundance of positively charged amino acids such as lysine or arginine or has sequences that contain an alternating pattern of polar/charged amino acid and non-polar, hydrophobic amino acids.

Examples of CPPs which can be used in the present invention include, without limitation, the CPP found in Drosophila antennapedia protein (RQIKIWFQNRRMKWKK. SEQ ID NO:13) , the CPP found in the herpesvirus simplex 1 (HSV-1) VP22 DNA-binding protein (DAATATRGRSAASRPTERPRAPARSASRPRRPVE, SEQ ID NO:14) , the CPP of Bac-7 (RRIRPRPPRLPRPRPRPLPFPRPG; SEQ ID NO: 15), the CPPs of the HIV-1 TAT protein consisting of amino acids 49-57 (RKKRRQRRR, SEQ ID NO: 16), amino acids 48-60 (GRKKRRQRRRTPQ, SEQ ID NO: 17), amino acids 47-57 (YGRKKRRQRRR; SEQ ID NO: 18); the CPP of S413-PV peptide (ALWKTLLKKVLKAPKKKRKV; SEQ ID NO: 19), the CPP of penetratin (RQIKWFQNRRMKWKK; SEQ ID NO: 20), the CPP of SynB1 (RGGRLSYSRRRFSTSTGR; SEQ ID NO: 21), the CPP of SynB3 (RRLSYSRRRF; SEQ ID NO: 22), the CPP of PTD-4 (PIRRRKKLRRLK; SEQ ID NO: 23), the CPP of PTD-5 (RRQRRTSKLMKR; SEQ ID NO: 24), the CPP of the FHV Coat-(35-49) (RRRRNRTRRNRRRVR; SEQ ID NO: 25), the CPP of BMV Gag-(7-25) (KMTRAQRRAAARRNRWTAR; SEQ ID NO: 26), the CPP of HTLV-II Rex-(4-16) (TRRQRTRRARRNR; SEQ ID NO: 27), the CPP of D-Tat (GRKKRRQRRRPPQ; SEQ ID NO:28), the CPP R9-Tat (GRRRRRRRRRPPQ; SEQ ID NO: 29), the CPP of MAP (KLALKLALKLALALKLA; SEQ ID NO: 30), the CPP of SBP (MGLGLHLLVLAAALQGAWSQPKKKRKV; SEQ ID NO: 31), the CPP of FBP (GALFLGWLGAAGSTMGAWSQPKKKRKV; SEQ ID NO: 32), the CPP of MPG (ac-GALFLGFLGAAGSTMGAWSQPKKKRKV-cya; SEQ ID NO: 33), the CPP of MPG(ENLS) (ac-GALFLGFLGAAGSTMGAWSQPKSKRKV-cya; SEQ ID NO: 34), the CPP of Pep-1 (ac-KETWWETWWTEWSQPKKKRKV-cya; SEQ ID NO: 35), the CPP of Pep-2 (ac-KETWFETWFTEWSQPKKKRKV-cya; SEQ ID NO: 36), a polyarginine sequence having the structure RN (wherein N is between 4 and 17), the GRKKRRQRRR sequence (SEQ ID NO: 37), the RRRRRRLR sequence (SEQ ID NO: 38), the RRQRRTS KLMKR sequence (SEQ ID NO: 39); Transportan GWTLNSAGYLLGKINLKALAALAKKIL (SEQ ID NO: 40); KALAWEAKLAKALAKALAKHLAKALAKALKCEA (SEQ ID NO: 41); RQIKIWFQNRRMKWKK (SEQ ID NO: 42), the YGRKKRRQRRR sequence (SEQ ID NO: 43); the RKKRRQRR sequence (SEQ ID NO: 44); the YARAAARQARA sequence (SEQ ID NO: 45); the THRLPRRRRRR sequence (SEQ ID NO: 46); the GGRRARRRRRR sequence (SEQ ID NO: 47).

In a preferred embodiment, said cell-penetrating peptide is not the endogenous contained in SEQ ID NO: 1.

In a preferred embodiment, the CPP is the CPP of the HIV-1 TAT protein consisting of amino acids 49-57 (RKKRRQRRR, SEQ ID NO: 16). In another preferred embodiment the CPP is the GRKKRRQRRR sequence (SEQ ID NO: 37) or RRRRRRLR (SEQ ID NO: 38). In another embodiment, the CPP is the GRKKRRQRRR sequence (SEQ ID NO: 37) or RRRRRRRR (SEQ ID NO: 65).

In some embodiments, a CPP is as a CPP as described in WO2019/018898, the content of which is incorporated herein by reference in its entirety.

In one embodiment, the cell-penetrating peptide sequence is fused at the N-terminus of the polypeptide of the invention or of the functionally equivalent variant of said polypeptide. In another embodiment, the cell-penetrating peptide is fused at the C-terminus of the polypeptide of the invention or of the functionally equivalent variant of said polypeptide.

In preferred embodiments, the conjugates or fusion proteins of the combination according to the invention comprise, in addition to the own cell penetrating peptide found in the polypeptide of SEQ ID NO: 1 or of the functionally equivalent variant of said polypeptide, at least 1, at least 2, at least 3, at least 4, at least 5, at least 6, at least 7, at least 8, at least 9, at least 10 or more additional cell penetrating peptides.

Suitable fusion proteins of the invention include the polypeptides Omomyc*TAT and Omomyc*LZArg as defined below:

| Name | SEQ ID NO: | Sequence |
|---|---|---|
| Omomyc^{∗}TAT | 11 | MTEENVKRRTHNVLERQRRNELKRSFFALRDQIPELENNEKAPKVVI LKKATAYILSVQAETQKLISEIDLLRKQNEQLKHKLEQLRNSCAGRK KRRQRRR |
| Omomyc^{∗}LZArg | 12 | MTEENVKRRTHNVLERQRRNELKRSFFALRDQIPELENNEKAPKVVI LKKATAYILSVQAETQKLISEIDLLRKQNEQLKHKLEQLRNSCARRR RRRLR |

Thus, in a preferred embodiment the fusion protein is the polypeptide selected from SEQ ID NO: 11 and 12.

Suitable assays for determining whether a conjugate preserves the cell membrane translocation capacity of Omomyc include, without limitation, assays which measure the capacity of the conjugate to transduce cells in culture. This assay is based on contacting the conjugate with culture cells and detecting the presence of the conjugate in an intracellular location.

In another preferred embodiment, the conjugate of the combination of the invention additionally comprises a further nuclear localization signal.

The term "nuclear localization signal" (NLS), as used herein, refers to an amino acid sequence of about 4-20 amino acid residues in length, which serves to direct a protein to the nucleus. Typically, the nuclear localization sequence is rich in basic amino acids and exemplary sequences are well known in the art (Gorlich D. (1998) EMBO 5.17:2721-7). In some embodiments, the NLS is selected from the group consisting of the SV40 large T Antigen NLS (PKKKRKV, SEQ ID NO: 48); the Nucleoplasmin NLS (KRPAATKKAGQAKKKK, SEQ ID NO: 49); the CBP80 NLS (RRRHSDENDGGQPHKRRK, SEQ ID NO: 50); the HIV-I Rev protein NLS (RQARRNRRRWE, SEQ ID NO: 51); the HTLV-I Rex (MPKTRRRPRRSQRKRPPT, SEQ ID NO: 52); the hnRNP A NLS (NQSSNFGPMKGGNFGGRSSGPYGGGGQYFKPRNQGGY, SEQ ID NO: 53); the rpL23a NLS (VHSHKKKKIRTSPTFTTPKTLRLRRQPKYPRKSAPRRNKLDHY, SEQ ID NO: 54). In one embodiment of the invention, the nuclear localization signal comprises the motif K (K/ R) X (K/ R).

In an even more preferred embodiment, the nuclear localization signal is selected from the group consisting of PKKKRKV (SEQ ID NO: 48), PAAKRVKLD (SEQ ID NO: 56) and KRPAATKKAGQ AKKKK (SEQ ID NO: 49).

In another preferred embodiment, the NLS may be N-terminal or C-terminal to the conjugate or the fusion protein comprising the polypeptide of SEQ ID NO: 1 or a functionally equivalent variant thereof.

The skilled person will understand that it may be desirable that the conjugate of the invention further comprises one or more flexible peptides that connect the polypeptide comprising SEQ ID NO: 1 or the functionally equivalent variant thereof, the cell penetrating peptide sequence and/or the NLS. Thus, in a particular embodiment the polypeptide comprising SEQ ID NO: 1 or a functionally equivalent variant thereof is directly connected to the cell penetrating peptide sequence. In another particular embodiment, the polypeptide comprising SEQ ID NO: 1 or a functionally equivalent variant thereof is connected to the cell penetrating peptide sequence through a flexible peptide. In an embodiment the polypeptide comprising SEQ ID NO: 1 or the functionally variant thereof is directly connected to the NLS. In another embodiment the polypeptide comprising SEQ ID NO: 1 or a functionally equivalent variant thereof is connected to the NLS through a flexible peptide.

In a particular embodiment the polypeptide of the conjugate according to the invention is directly connected to the cell penetrating peptide sequence and to the NLS.

In one embodiment, the NLS is one of the NLS which appears endogenously in the Myc sequence, such as the M1 peptide (PAAKRVKLD, SEQ ID NO: 56) or the M2 peptide (RQRRNELKRSF, SEQ ID NO: 57).

In another embodiment the additional NLS refers to an NLS which is different to the endogenous NLS found in a polypeptide comprising SEQ ID NO: 1 or in the functionally equivalent variant of SEQ ID NO: 1.

In preferred embodiments, the conjugates or fusion proteins according to the invention comprise, in addition to the endogenous NLS found in the polypeptide of the invention or in the functionally equivalent variant thereof, at least 1, at least 2, at least 3, at least 4, at least 5, at least 6, at least 7, at least 8, at least 9, at least 10 NLS.

In another particular embodiment, the polypeptide of the conjugate fore use according to the invention is connected to the cell penetrating peptide sequence through a first flexible peptide linker and to the NLS through a second flexible peptide linker.

As used herein, the term "flexible peptide", "spacer peptide" or "linker peptide" refers to a peptide that covalently binds two proteins or moieties but which is not part of either polypeptide, allowing movement of one with respect to the other, without causing a substantial detrimental effect on the function of either the protein or the moiety. Thus, the flexible linker does not affect the tumour tracing activity of the polypeptide sequence, the cell penetrating activity of the cell penetrating peptide or the nuclear localization capacity of the NLS.

The flexible peptide comprises at least one amino acid, at least two amino acids, at least three amino acids, at least four amino acids, at least five amino acids, at least six amino acids, at least seven amino acids, at least eight amino acids, at least nine amino acids, at least 10 amino acids, at least 12 amino acids, at least 14 amino acids, at least 16 amino acids, at least 18 amino acids, at least 20 amino acids, at least 25 amino acids, at least 30 amino acids, at least 35 amino acids, at least 40 amino acids, at least 45 amino acids, at least 50 amino acids, at least 60 amino acids, at least 70 amino acids, at least 80 amino acids, at least 90 amino acids, or about 100 amino acids. In some embodiments the flexible peptide will permit the movement of one protein with respect to the other in order to increase solubility of the protein and/or to improve its activity. Suitable linker regions include a poly-glycine region, the GPRRRR sequence (SEQ ID NO: 58) of combinations of glycine, proline and alanine residues.

In a particular embodiment, the conjugates according to the invention comprise a tag bound to the conjugate or to the C-terminal or N-terminal domain of said polypeptide or fusion protein or variant thereof. Said tag is generally a peptide or amino acid sequence which can be used in the isolation or purification of said fusion protein. Thus, said tag is capable of binding to one or more ligands, for example, one or more ligands of an affinity matrix such as a chromatography support or bead with high affinity. An example of said tag is a histidine tag (His-tag or HT), such as a tag comprising 6 residues of histidine (His6 or H6), which can bind to a column of nickel (Ni²⁺) or cobalt (Co²⁺) with high affinity. His-tag has the desirable feature that it can bind its ligands under conditions that are denaturing to most proteins and disruptive to most protein-protein interactions. Thus, it can be used to remove the bait protein tagged with H6 following the disruption of protein-protein interactions with which the bait has participated.

Additional illustrative, non-limitative, examples of tags useful for isolating or purifying the conjugate or the polypeptide comprising SEQ ID NO: 1 or a variant thereof or a fusion protein include Arg-tag, FLAG-tag (DYKDDDDK; SEQ ID NO:59), Strep-tag (WSHPQFEK, SEQ ID NO:60), an epitope capable of being recognized by an antibody, such as c-myc-tag (recognized by an anti-c-myc antibody), HA tag (YPYDVPDYA, SEQ ID NO:61), V5 tag (GKPIPNPLLGLDST, SEQ ID NO:62), SBP-tag, S-tag, calmodulin binding peptide, cellulose binding domain, chitin binding domain, glutathione S-transferase-tag, maltose binding protein, NusA, TrxA, DsbA, Avi-tag, etc. (Terpe K., Appl. Microbiol. Biotechnol. 2003, 60:523-525), an amino acid sequence such as AHGHRP (SEQ ID NO:63) or PIHDHDHPHLVIHSGMTCXXC (SEQ ID NO:64), β-galactosidase and the like.

The tag can be used, if desired, for the isolation or purification of said fusion protein.

In another preferred embodiment, the anti-cancer treatment is a polynucleotide encoding the polypeptide or the fusion protein disclosed above. In a preferred embodiment, the anti-cancer treatment is a polynucleotide encoding a polypeptide comprising the sequence SEQ ID NO: 1 or a functionally equivalent variant thereof. In another embodiment, the anti-cancer treatment is a polynucleotide encoding a conjugate comprising the polypeptide comprising the sequence SEQ ID NO: 1 or a functionally equivalent variant thereof and a chemical moiety that facilitates cellular uptake of the polypeptide or of the functionally equivalent variant thereof; more preferably is a polynucleotide encoding a fusion protein between the polypeptide comprising the sequence SEQ ID NO: 1 or a functionally equivalent variant thereof and a cell-penetrating peptide sequence.

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a cell.

In preferred embodiment, the polynucleotide of the invention is an mRNA.

mRNA can be chemically synthesized, can be obtained by means of *in vitro* transcription or can be synthesized *in vivo* in the target cell. The nucleotide sequences that form the polynucleotide encoding the conjugate or fusion protein of the invention are in the same correct reading frame for expression thereof.

In a preferred embodiment, the anti-cancer treatment is an mRNA encoding for a polypeptide consisting of the sequence SEQ ID NO: 1 or a polypeptide consisting of a functionally equivalent variant of SEQ ID NO: 1 or a polypeptide consisting of SEQ ID NO: 4.

In another embodiment, the anti-cancer treatment is a vector comprising a polynucleotide of the invention.

The term "vector", as used herein, refers to a nucleic acid sequence comprising the necessary sequences so that after transcribing and translating said sequences in a cell a polypeptide encoded by the polynucleotide of the invention is generated. Said sequence is operably linked to additional segments that provide for its autonomous replication in a host cell of interest. Preferably, the vector is an expression vector, which is defined as a vector which, in addition to the regions of the autonomous replication in a host cell, contains regions operably linked to the nucleic acid of the invention and which are capable of enhancing the expression of the products of the nucleic acid according to the invention. The vectors of the invention can be obtained by means of techniques widely known in the art.

Examples of vectors include, but are not limited to, viral vectors, naked DNA or RNA expression vectors, plasmid, cosmid or phage vectors, DNA or RNA expression vectors associated with cationic condensing agents, DNA or RNA expression vectors encapsulated in liposomes, and certain eukaryotic cells, such as producer cells. Suitable vectors comprising a polynucleotide of the invention are vectors derived from expression vectors in prokaryotes such as pUC18, pUC19, pBluescript and their derivatives, mp18, mp19, pBR322, pMB9, ColE1, pCRI, RP4, phages and "shuttle" vectors such as pSA3 and pAT28, expression vectors in yeasts such as vectors of the 2-micron plasmid type, integration plasmids, YEP vectors, centromeric plasmids and similar, expression vectors in insect cells such as the vectors of the pAC series and of the pVL series, expression vectors in plants such as vectors of the series pIBI, pEarleyGate, pAVA, pCAMBIA, pGSA, pGWB, pMDC, pMY, pORE and similar and expression vectors in superior eukaryote cells based on viral vectors (adenovirus, virus associated to adenovirus as well as retrovirus and, in particular, lentivirus) as well as non-viral vectors such as pSilencer 4.1-CMV (Ambion), pcDNA3, pcDNA3.1/hyg, pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL, pKSV-10, pBPV-1, pML2d and pTDT1. In a preferred embodiment, the polynucleotide of the invention is comprised in a vector selected from the group consisting of pEGFP or pBabe retroviral vectors and pTRIPZ or pSLIK lentiviral vectors.

The vector of the invention may be used to transform, transfect or infect cells that can be transformed, transfected or infected by said vector. Said cells may be prokaryotic or eukaryotic.

The vector preferably comprises the polynucleotide of the invention operationally bound to sequences that regulate the expression of the polynucleotide of the invention. The regulatory sequences of use in the present invention may be nuclear promoters or, alternatively, enhancer sequences and/or other regulatory sequences that increase expression of the heterologous nucleic acid sequence. In principle, any promoter can be used in the present invention provided said promoter is compatible with the cells wherein the polynucleotide is to be expressed. Thus, promoters suitable for realizing the present invention include, but are not necessarily limited to, constitutive promoters such as derivatives of eukaryotic virus genomes such as polyoma virus, adenovirus, SV40, CMV, avian sarcoma virus, hepatitis B virus, the metallothionein gene promoter, the herpes simplex virus thymidine kinase gene promoter, LTR regions of retroviruses, the immunoglobulin gene promoter, the actin gene promoter, the EF-1alpha gene promoter as well as inducible promoters wherein protein expression depends on the addition of a molecule or exogenous signal, such as tetracycline systems, the NFκB/UV light system, the Cre/Lox system and the heat shock genes promoter, the regulable RNA polymerase II promoters described in WO/2006/135436 and tissue-specific promoters.

In another embodiment, the anti-cancer treatment is a cell capable of secreting into the medium the polypeptide of the invention or the conjugate of the invention, preferably the polypeptide of the invention or the fusion protein of the invention.

Suitable cells capable of secreting a polypeptide of the invention include without limitation, cardiomyocytes, adipocytes, endothelial cells, epithelial cells, lymphocytes (B and T cells), mastocytes, eosinophils, vascular intima cells, primary cultures of isolated cells of different organs, preferably of cells isolated from Langerhans islets, hepatocytes, leukocytes, including mononuclear leukocytes, mesenchymal, umbilical cord or adult (of skin, lung, kidney and liver), osteoclasts, chondrocytes and other connective tissue cells. Cells of established lines such as Jurkat T cells, NIH-3T3, CHO, Cos, VERO, BHK, HeLa, COS, MDCK, 293, 3T3 cells, C2C12 myoblasts and W138 cells are also suitable. Persons skilled in the art will appreciate that the cells capable of secreting into the medium a polypeptide of the invention may be found forming microparticles or microcapsules so that the cells have a greater useful life in patients. Materials suitable for the formation of microparticles object of the invention include any biocompatible polymeric material which permits continuous secretion of the therapeutic products and which acts as support of the cells. Thus, said biocompatible polymeric material may be, for example, thermoplastic polymers or hydrogen polymers. Among the thermoplastic polymers we have acrylic acid, acrylamide, 2-aminoethyl methacrylate, poly(tetrafluoroethylene-cohexafluorpropylene), methacrylic-(7-cumaroxy) ethyl ester acid, N-isopropyl acrylamide, polyacrylic acid, polyacrylamide, polyamidoamine, poly(amino)-p-xylylene, poly(chloroethylvinylether), polycaprolactone, poly(caprolactone-co-trimethylene carbonate), poly(carbonate urea) urethane, poly(carbonate) urethane, polyethylene, polyethylene and acrylamide copolymer, polyethylene glycol, polyethylene glycol methacrylate, poly(ethylene terephthalate), poly(4-hydroxybutyl acrylate), poly(hydroxyethyl methacrylate), poly(N-2-hydroxypropyl methacrylate), poly(lactic glycolic acid), poly(L-lactic acid), poly(gamma-methyl, Lglutamate), poly(methylmethacrylate), poly(propylene fumarate), poly(propylene oxide), polypyrrole, polystyrene, poly(tetrafluoroethylene), polyurethane, polyvinyl alcohol, polyethylene of ultra-high molecular weight, 6-(p-vinylbenzamide)-hexanoic acid N-pvinybenzyl-D-maltonamide and copolymers containing more than one of said polymers. Among the polymers of hydrogel type we have natural materials of alginate, agarose, collagen, starch, hyaluronic acid, bovine serum albumin, cellulose and their derivatives, pectin, chondroitin sulphate, fibrin and fibroin, as well as synthetic hydrogels such as Sepharose^{®} and Sephadex^{®}.

The method for predicting the clinical response of the invention is carried out in a pretreatment sample, i.e., in a sample of the patient obtained before the patient has received the treatment whose response is predicted.

Regarding the treatment received by the subject before the isolation of the sample in any of the aspects of the invention, preferably the subject has not received any anti-cancer treatment at any time, preferably within the last 168 hours, more preferably within the last 72 hours, more preferably within the last 48 hours, even more preferably within the last 36 hours, yet more preferably within the last 24 hours, even yet more preferably at 24 hours prior to the isolation of the sample. In another preferred embodiment, the subject has not received an anti-cancer treatment 24 hours prior to the isolation of the sample, but preferably within the last 23 hours, preferably the last 22 hours, more preferably within the last 21 hours, yet more preferably within the last 20 hours, even more preferably within the last 15 hours, even yet more preferably within the last 10 hours, even more preferably within the last 5 hours, and even yet more preferably within the last 2 hours, yet even more preferably within the last hour prior to the isolation of the sample. In yet a more preferred embodiment, the subject from whom the sample has been isolated has not received any anti-cancer treatment at any time before the isolation of the sample, i.e., the subject has never received an anticancer treatment.

In a preferred embodiment, the subject has not received any anti-cancer treatment within the last 24 hours prior to the isolation of the sample.

In another particular embodiment, the subject from whom the sample is isolated has received an anti-cancer treatment at any time in a period prior to the isolation of the sample, preferably within the last 168 hours, more preferably within the last 72 hours, even more preferably within the last 48 hours, even more preferably within the last 36 hours, yet more preferably within the last 24 hours prior to the isolation of the sample. If administered at 24 hours prior to the isolation of the sample, preferably within the last 24 hours prior to the isolation of the sample, said anti-cancer treatment is different from an anti-cancer treatment selected from the group consisting of items (a) to (e) of the first aspect of the invention.

In a preferred embodiment, the subject from whom the sample has been isolated has received an anti-cancer treatment before the isolation of the sample, and wherein the anti-cancer treatment is different from a treatment selected from the group consisting of items (a) to (e) of the first aspect of the invention. Preferably, said anticancer treatment is selected from surgery, chemotherapy, radiation therapy, hormonal therapy and targeted therapy, including immunotherapy, and combinations thereof. In a preferred embodiment said anticancer therapy is selected from the group consisting of surgery and radiation therapy. In a more preferred embodiment said anticancer therapy is radiation therapy.

In a preferred embodiment, the subject from whom the sample is isolated has not received systemic anti-cancer treatment within at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 20 weeks, at least 30 weeks, at least 40 weeks, at least 50 weeks prior to the isolation of the sample. In a more preferred embodiment the subject has not received systemic anti-cancer treatment within at least 4 weeks prior to the isolation of the sample.

The moment after the initiation of therapy in which the response is evaluated is no particularly limited. Thus, response to therapy can be determined at least after 1, 2, 3, 4, 5, 6, 7, 8, 9, 10, 11, 12, 13, 14, 15, 16, 17 ,18, 19 or 20 cycles of therapy. Response to therapy can be determined at least 1, 2, 3, 4, 5, 6, 7, 8 9, 10 or 11 months after the initiation of the first therapy or at least 1, 2, 3, 4, 5, 6, 7, 8, 9, 10 or more years after the initiation of the first therapy. In a particular embodiment response to therapy can be assessed wherein the subject has received at least two, preferably at least three prior lines of anti-cancer treatment.

In addition, the type and number of lines of therapies applied to the patient before the determination of the response is also not particularly limited. Thus, the response can be determined in patients after the patient has been treated with a first line of therapy including surgical therapy, chemotherapy or surgery in combination with adjuvant or neoadjuvant chemotherapy or any type of therapy , or after the patient has been treated with two or more sequential or simultaneous lines of therapy.

Thus, the first step of the first method of the invention comprises determining the level of at least one biomarker selected from the group consisting of MIP-1β, CD62E, IL-8, GM-CSF and IL-1α in a sample from a subject suffering from cancer whose response to treatment is to be predicted.

The levels of these biomarkers can be detected in any kind of sample. The term "sample", as used herein, relates to any sample which can be obtained from the subject and which contains any biological material suitable for detecting RNA or protein levels. In a particular embodiment, the sample contains genetic material, e.g., DNA, genomic DNA (gDNA), complementary DNA (cDNA), RNA, heterogeneous nuclear RNA (hnRNA), mRNA, etc., from the subject under study. In another particular embodiment the sample contains proteins. The sample can comprise cell and/or non-cell material of the subject. The present method can be applied to any kind of biological sample from a subject, such as a biopsy sample, tissue, cell or biological fluid (blood, plasma, serum, saliva, urine, semen, sputum, cerebral spinal fluid (CSF), tears, mucus, sweat, milk), feces, brain extracts, bone marrow, nipple aspirate, samples obtained by bronchial lavage, bronchoscopy, fine needle aspiration biopsy (FNAB), solid tumor biopsy sample, a buccal or buccal pharyngeal swab and the like. Said sample can be obtained by conventional methods, e.g., biopsy, surgical excision or aspiration, by using methods well known to those of ordinary skill in the related medical arts. Methods for obtaining the sample from the biopsy include gross apportioning of a mass, or microdissection or other art-known cell-separation methods. Tumor cells can additionally be obtained from fine needle aspiration cytology. In a preferred embodiment samples are obtained by fine needle aspiration biopsy (FNAB). In order to simplify conservation and handling of the samples, these can be formalin-fixed and paraffin-embedded or first frozen and then embedded in a cryosolidifiable medium, such as OCT-Compound, through immersion in a highly cryogenic medium that allows rapid freeze. The samples can also be a cell suspension, cell pellet, cell slide, frozen solid tumor biopsy.

In a particular embodiment, the sample is tumor tissue sample, preferably a biopsy of a tumor tissue sample, either fresh or frozen.

In a particular embodiment, said sample comprises cancer cells, preferably breast cancer, ovarian cancer, prostate cancer, gastric cancer, pancreatic cancer, lung cancer, colorectal cancer, stomach/gastric cancer, endometrial/uterine/cervical cancer, bladder cancer, head and neck cancer, leukemia, sarcoma, cholangiocarcinoma, glioblastoma, multiple myeloma, lymphoma cells. In a preferred embodiment, the sample comprises cells selected from the group consisting of pancreatic cancer cells, lung cancer cells, colorectal cancer cells, salivary gland cells and sarcoma cells. In a preferred embodiment it is a tumor tissue sample or portion thereof. Preferably, said tumor tissue sample is a breast tumor, ovarian tumor, prostate tumor, gastric tumor, pancreatic tumor, lung tumor, colorectal tumor, stomach/gastric tumor, endometrial/uterine/cervical tumor, bladder tumor, head and neck tumor, sarcoma tumor, cholangiocarcinoma tumor, glioblastoma tumor, multiple myeloma tumor, lymphoma tumor tissue, or portion thereof, sample. In a preferred embodiment, the sample is a pancreatic tumor sample, a lung tumor sample, a colorectal tumor sample, a salivary gland tumor sample or a sarcoma tumor sample.

In another particular embodiment, the sample from the subject according to the methods of the invention is a biofluid, preferably a biofluid from affected organs. A "biofluid", "biological fluid sample" or "bodily fluid sample", as used herewith, refers to any biological secretion or fluid, whether physiological or pathological, which is produced in the body of a subject. Such biofluids include, without limitation, blood, plasma, serum, bronchoalveolar washing fluid, urine, nasal secretion, ear secretion, urethral secretion, cerebrospinal fluid, pleural fluid, synovial fluid, peritoneal fluid, ascites fluid, pericardial liquid, amniotic fluid, gastric juice, lymphatic fluid, interstitial fluid, saliva, sputum, liquid deposition, tears, mucus, sweat, milk, semen, vaginal secretions, fluid coming from ulcer, blisters, abscesses and other surface eruptions. Said samples can be obtained by conventional methods, using processes known in the state of art by the person skilled in the art, such as blood extraction, instillation and aspiration of liquid during bronchofibroscopy, cisternal, ventricular or lumbar puncture, pleural puncture or thoracocentesis, joint or synovial percutaneous puncture, abdominal puncture, amniocentesis, expectoration, peritoneal percutaneous puncture, pericardial percutaneous puncture, etc., or by simple harvesting.

In a preferred embodiment, the sample is selected from the group consisting of blood, serum, plasma, saliva, cerebrospinal fluid (CSF) and a tumor biopsy sample; more preferably is selected from the group consisting of blood, plasma and serum; even more preferably is serum.

The blood sample is typically extracted by means of puncturing an artery or vein, normally a vein from the inner part of the elbow or from the back of the hand, the blood sample being collected in an air-tight vial or syringe. A capillary puncture normally on the heel or on the distal phalanxes of fingers can be performed for analysis by means of a micromethod. Serum can be obtained from the complete blood sample and in the absence of anticoagulant by leaving the sample to settle for 10 minutes so that it coagulates and subsequently centrifuging it at 1,500 rpm for 10 minutes for the purpose of separating the cells (precipitate) from the serum (supernatant). In turn, to obtain the plasma sample the complete blood is contacted with an anticoagulant and is centrifuged at 3,000 rpm for 20 minutes. The precipitate of said centrifugation corresponds to the formed elements, and the supernatant corresponds to the plasma. The serum or the plasma obtained can be transferred to a storage tube for sample analysis by means of the method of the invention.

The term "biomarker", as used herein, refers to a gene product from a gene of interest, understood as the transcriptional product or the translational product of a gene of interest (i.e. the mRNA transcribed from said gene or the protein encoded by said gene), the amount of which reflects a specific situation, for example, a condition such as the prediction of the response of a subject suffering from cancer to an anti-cancer treatment.

The biomarkers of the invention are primarily (i.e. the primary biomarkers of the invention are) MIP-1β, CD62E, IL-8, GM-CSF and IL-1α.

The term "MIP-1β", "MIP-1b", "MIP1B" or "MIP-1beta" refers to "macrophage inflammatory protein-1beta", and is now officially named as "CCL4" or "chemokine (C-C motif) ligand 4" or "C-C motif chemokine 4". Older names for human CCL4 are also AT 744, Act-2, LAG-1, HC21 and G-26. As used herein, this term refers to the protein or the gene encoding the protein with UniProt accession number P13236 (version 216 of the entry as of 12 October 2022). The term "C-C motif chemokine 4" includes any of the isoforms that have been described for this protein.

The term "CD62E" refers to "CD62 antigen-like family member E", and is also known as "SELE", "ELAM1" "ELAM-1", "LECAM2", "E-selectin", "endothelial-leukocyte adhesion molecule 1" or "leukocyte-endothelial cell adhesion molecule 2". As used herein, this term refers to the protein or the gene encoding the protein with UniProt accession number P16581 (version 235 of the entry as of 12 October 2022). The term "CD62 antigen-like family member E" includes any of the isoforms that have been described for this protein.

The term "IL-8" or "CXCL8" refers to "interleukin-8" or "chemokine (C-X-C motif) ligand 8". As used herein, this term refers to the protein or the gene encoding the protein with UniProt accession number P10145 (version 237 of the entry as of 12 October 2022). The term "interleukin-8" includes any of the isoforms that have been described for this protein.

The term "GM-CSF" or "GMCSF" refers to "granulocyte-macrophage colony-stimulating factor", also known as "CSF2" or "colony-stimulating factor 2" and, alternatively as "molgramostin" or "sargramostim". As used herein, this term refers to the protein or the gene encoding the protein with UniProt accession number P04141 (version 207 of the entry as of 12 October 2022). The term "granulocyte-macrophage colony-stimulating factor" includes any of the isoforms that have been described for this protein.

The term "IL-1a", "IL-1a", "IL-1 alpha", "IL1A" or "IL1F1" refers to "interleukin-1 alpha", also named as "hematopoietin-1". As used herein, this term refers to the protein or the gene encoding the protein with UniProt accession number P01583 (version 224 of the entry as of 12 October 2022). The term "interleukin-1 alpha" includes any of the isoforms that have been described for this protein.

The expression "at least one biomarker selected from the group consisting of MIP-1β, CD62E, IL-8, GM-CSF and IL-1a", as used herein, means that according to the present invention, the level of expression of one, two, three, four or five of said biomarkers can be determined. In a particular embodiment, the method comprises the determination of the level of expression of one of said biomarkers. In a preferred embodiment the biomarker determined is MIP-1β. In another embodiment the biomarker determined is CD62E. In another embodiment the biomarker determined is IL-8. In another embodiment the biomarker determined is GM-CSF. In another embodiment the biomarker determined is IL-1α. In another particular embodiment, the method of the invention comprises determining the level of expression of two of said biomarkers. In a preferred embodiment the biomarkers determined are MIP-1β and CD62E. In another embodiment the biomarkers determined are MIP-1β and IL-8. In another embodiment the biomarkers determined are MIP-1β and GM-CSF. In another embodiment the biomarkers determined are MIP-1β and IL-1α. In another embodiment the biomarkers determined are CD62E and IL-8. In another embodiment the biomarkers determined are CD62E and GM-CSF. In another embodiment the biomarkers determined are CD62E and IL-1α. In another embodiment the biomarkers determined are IL-8 and GM-CSF. In another embodiment the biomarkers determined are IL-8 and IL-1α. In another embodiment the biomarkers determined are GM-CSF and IL-1α. In another embodiment the method of the invention comprises determining the level of expression of three of said biomarkers. In another embodiment the method of the invention comprises determining the level of expression of four of said biomarkers. In another particular embodiment the method of the invention comprises determining the level of expression of the five biomarkers MIP-1β, CD62E, IL-8, GM-CSF and IL-1α.

Any combination of primary biomarkers is encompassed by the methods of the invention.

In a more preferred embodiment, the method comprises determining the levels of a combination of biomarkers selected from the group consisting of: a) a combination of biomarkers comprising MIP-1β and CD62E; and b) a combination of biomarkers comprising CD62E and IL-8. More preferably, the combination is selected from the group consisting of: a) a combination of biomarkers consisting of MIP-1β and CD62E; and b) a combination of biomarkers consisting of CD62E and IL-8.

The method of the invention may further comprise the determination of other biomarkers different from MIP-1β, CD62E, IL-8, GM-CSF, and IL-1α.

Therefore, in a preferred embodiment, the method further comprises the determination of the level of expression of additional biomarkers different from the primary biomarkers of the invention (i.e., secondary biomarkers), preferably the method further comprises determining the level of at least one additional biomarker selected from the group consisting of MCP-1, ICAM-1, IFN-gamma, IL-1β, IL-12 and combinations thereof.

The term "MCP-1" or "MCP1" refers to "monocyte chemoattractant protein 1" or "monocyte chemotactic protein 1", and is also known as "CCL2" or "chemokine (C-C motif) ligand 2" or "C-C motif chemokine 2" or "small inducible cytokine A2" or "SCYA2". As used herein, this term refers to the protein or the gene encoding the protein with UniProt accession number P13500 (version 246 of the entry as of 12 October 2022). The term "monocyte chemoattractant protein 1" includes any of the isoforms that have been described for this protein.

The term "ICAM-1" or "ICAM1" refers to "intercellular adhesion molecule 1", also known as "CD54" or "cluster of differentiation 54". As used herein, this term refers to the protein or the gene encoding the protein with UniProt accession number P05362 (version 251 of the entry as of 12 October 2022). The term "intercellular adhesion molecule 1" includes any of the isoforms that have been described for this protein.

The term "IFN-gamma", "IFN-g", "IFN-γ" or "IFNG" refers to "interferon gamma". As used herein, this term refers to the protein or the gene encoding the protein with UniProt accession number P01579 (version 238 of the entry as of 12 October 2022). The term "interferon gamma" includes any of the isoforms that have been described for this protein.

The term "IL-1β", "IL-1b", "IL-1 beta", "IL1beta", "IL1B" or "IL1F2" refers to "interleukin-1 beta", also known as "leukocytic pyrogen", "leukocytic endogenous mediator", "mononuclear cell factor", or "lymphocyte activating factor". As used herein, this term refers to the protein or the gene encoding the protein with UniProt accession number P01584 (version 249 of the entry as of 12 October 2022). The term "interleukin-1 beta" includes any of the isoforms that have been described for this protein.

The term "IL-12" refers to "interleukin 12". IL-12 is a heterodimeric cytokine encoded by two separate genes: IL-12A (p35) and IL-12B (p40). The active heterodimer (referred to as "p70") and a homodimer of p40 are formed following protein synthesis. As used herein, this term refers to any of the proteins or the genes encoding the proteins with UniProt accession number P29459 (version 190 of the entry as of 12 October 2022) and P29460 (version 231 of the entry as of 12 October 2022). The protein with UniProt accession number P29459 relates to "interleukin-12 subunit alpha" or "IL12A" or "IL-12A" or "NKSF1". The protein with UniProt accession number P29460 relates to "interleukin-12 subunit beta" or "IL12B" or "NKSF2".

In a preferred embodiment the method of the invention further comprises determining the level of expression of MCP-1. In another embodiment, the method further comprises determining the level of expression of ICAM-1. In another embodiment, the method further comprises determining the level of expression of IFN-gamma. In another embodiment, the method further comprises determining the level of expression of IL-1β. In another embodiment, the method further comprises determining the level of expression of IL-12. In another embodiment the method of the invention further comprises determining the level of expression of a combination of MCP-1, ICAM-1, IFN-gamma, IL-1β, IL-12, preferably the level of expression of MCP-1, ICAM-1, IFN-gamma, IL-1β and IL-12.

Any combination of secondary biomarkers is encompassed by the methods of the invention.

In a preferred embodiment, the method of the invention comprises determining the levels of a combination of biomarkers selected from the group consisting of: a) a combination of biomarkers comprising MIP-1β and MCP-1; b) a combination of biomarkers comprising MIP-1β and ICAM-1; c) a combination of biomarkers comprising MIP-1β and CD62E; d) a combination of biomarkers comprising MIP-1β and IFN-gamma; e) a combination of biomarkers comprising MIP-1β and IL-1β; f) a combination of biomarkers comprising MIP-1β and IL-12; and g) a combination of biomarkers comprising CD62E and IL-8. Preferably, the method comprises determining the level of a combination of biomarkers selected from the group consisting of: a) a combination of biomarkers comprising MIP-1β and MCP-1; and b) a combination of biomarkers comprising CD62E and IL-8. In a preferred embodiment the combination of biomarkers consists of the combinations defined above.

In a preferred embodiment, the method of the first aspect of the invention further comprises:
i) determining the level of at least one additional biomarker selected from the group consisting of MCP-1, ICAM-1, IFN-gamma, IL-1β and IL-12 in a sample from the subject; and
ii) comparing the level of said at least one biomarker with a reference value, wherein:
   - a decreased level of said at least one biomarker with respect to the reference value is indicative of a good clinical response of the subject to the anti-cancer treatment, or
   - an equal or an increased level of said at least one biomarker with respect to the reference value is indicative of a poor clinical response of the subject to the anti-cancer treatment.

Any combination of primary and secondary biomarkers is encompassed by the methods of the invention.

The term "level" is also understood as "expression level" or "level of expression" and, as used herein, refers to the expression level of a gene product, more specifically to a measurable quantity of a gene product produced by a specific gene in a specific sample of the subject. The term "gene product", as used herein, refers to a transcriptional product or a translational product, and thus corresponds to the mRNA transcribed from said gene or to the protein encoded by a specific gene. When referring to the expression level of a biomarker, it is understood that the gene product is said biomarker, which, as indicated below, can be an mRNA or a protein transcribed from or encoded by the selected genes of the invention. As understood by the person skilled in the art, the gene expression level can be quantified by measuring the messenger RNA levels of said gene or of the protein encoded by said gene. In the context of the present invention, the expression level of the gene encoding MIP-1β, CD62E, IL-8, GM-CSF, IL-1a, MCP-1, ICAM-1, IFN-gamma, IL-1β or IL-12 can be determined by measuring the levels of mRNA encoded by said genes, or by measuring the levels of the protein encoded by said genes, i.e. MIP-1β, CD62E, IL-8, GM-CSF and IL-1a, MCP-1, ICAM-1, IFN-gamma, IL-1β or IL-12 protein or of variants thereof. MIP-1β, CD62E, IL-8, GM-CSF, IL-1a, MCP-1, ICAM-1, IFN-gamma, IL-1β and IL-12 protein variants include all the physiologically relevant post-translational chemical modifications forms of the protein, for example, glycosylation, phosphorylation, acetylation, etc., provided that the functionality of the protein is maintained. Said term encompasses the MIP-1β, CD62E, IL-8, GM-CSF, IL-1a, MCP-1, ICAM-1, IFN-gamma, IL-1β and IL-12 protein of any mammal species, including but not being limited to domestic and farm animals (cows, horses, pigs, sheep, goats, dogs, cats or rodents), primates and humans. Preferably, the MIP-1β, CD62E, IL-8, GM-CSF, IL-1a, MCP-1, ICAM-1, IFN-gamma, IL-1β and IL-12 protein is a human protein.

As the person skilled in the art understands, the expression levels of said biomarkers can be measured by determining the mRNA expression levels of the genes encoding said biomarkers or by determining the protein levels encoded by said genes.

In a preferred embodiment, the levels of at least one biomarker that are determined are mRNA levels.

In order to measure the mRNA levels of said biomarkers, the biological sample may be treated to physically, mechanically or chemically disrupt tissue or cell structures, to release intracellular components into an aqueous or organic solution to prepare nucleic acids for further analysis. The nucleic acids are extracted from the sample by procedures known to the skilled person using commercially available reagents. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained from fresh tumor tissue or from OCT embedded frozen tissue.

Suitable methods to determine gene expression levels at the mRNA level include, without limitation, standard assays for determining mRNA expression levels such as qPCR, RT-PCR, RNA protection analysis, Northern blot, RNA dot blot, TaqMan^{®}, tag based methods such as serial analysis of gene expression (SAGE) including variants such as LongSAGE and SuperSAGE, microarrays, nucleic acid sequence based amplification (NASBA), Fluorescence In Situ Hybridization, including variants such as Flow-FISH, qFiSH and double fusion fish (D-FISH), and the like.

In a particular embodiment the expression levels of the biomarkers are determined by a microarray. In another particular embodiment, the expression levels of the biomarkers are determined by quantitative PCR, preferably Real-Time PCR.

In order to normalize the values of mRNA expression among the different samples, it is possible to compare the expression levels of the mRNA of interest in the test samples with the expression of a control RNA. A "control RNA", as used herein, relates to a RNA whose expression levels do not change or change only in limited amounts in tumour cells with respect to non-tumorigenic cells. Preferably, the control RNA is mRNA derived from housekeeping genes and which code for proteins which are constitutively expressed and carry out essential cellular functions. Examples of housekeeping genes for use in the present invention include β-2-microglobulin, ubiquitin, 18-S ribosomal protein, cyclophilin, GAPDH, PSMB4, tubulin and β-actin.

In one embodiment relative gene expression quantification is calculated according to the comparative threshold cycle (Ct) method using GAPDH, β-actin or PSMB4 as an endogenous control and commercial RNA controls as calibrators. Final results are determined according to the formula 2-(ΔCt sample-ΔCt calibrator), where ΔCT values of the calibrator and sample are determined by substracting the CT value of the target gene from the value of the control gene.

In a preferred embodiment the levels of at least one biomarker that are determined are protein levels.

The expression levels of said biomarkers may also be measured by determining the protein levels or variants thereof encoded by the genes. Practically any conventional method can be used within the context of the present invention to quantify the levels of said proteins. Suitable methods to determine gene expression levels at the protein level include, without limitation, convention methods such as using antibodies with a capacity to specifically bind to the proteins encoded by said genes (or to fragments thereof containing antigenic determinants) and subsequent quantification of the resulting antibody-antigen complexes. In a particular embodiment, the protein levels of the biomarkers of the invention can be quantified by using standard assays for determining protein expression levels such as Western-blot or Western transfer, immunoassays such as ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of protein biochips or microarrays which include specific antibodies or assays based on colloidal precipitation in formats such as dipsticks or immunoassays based on luminex technology.

The antibodies to be employed in these assays can be, for example, polyclonal sera, hybridoma supernatants or monoclonal antibodies, antibody fragments, Fv, Fab, Fab' and F(ab')2, ScFv, diabodies, triabodies, tetrabodies and humanized antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc. There are a wide variety of well-known assays that can be used in the present invention, which use non-labeled antibodies (primary antibody) and labeled antibodies (secondary antibodies); among these techniques are included Western blot or Western transfer, ELISA, RIA, competitive EIA, DAS-ELISA, immunocytochemical and immunohistochemical techniques, techniques based on the use of biochips or protein microarrays including specific antibodies or assays based on colloidal precipitation in formats such as dipsticks. Other ways of detecting and quantifying the levels of the protein of interest include techniques of affinity chromatography, binding-ligand assays, etc.

On the other hand, the determination of the levels of the biomarker proteins can be carried out by constructing a tissue microarray (TMA) containing the subject samples assembled, and determining the expression levels of the corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumor cells and the specific cut-off for each marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol.2009; 47: 349-54) or Mulrane, L. et al. (Expert Rev. Mol. Diagn. 2008; 8: 707-25).

Alternatively, in another particular embodiment, the levels of the biomarker proteins are determined by Western blot. Western blot is based on the detection of proteins previously resolved by gel electrophoresis under denaturing conditions and immobilized on a membrane, generally nitrocellulose, by the incubation with an antibody specific and a developing system (e.g. chemoluminiscent).

In a particular embodiment, the expression levels are determined as protein levels. In a more particular embodiment, the protein levels are determined by ELISA, Western blot or an immunoassay.

In a preferred embodiment, the levels are determined by an immunoassay. The term "immunoassay", as used herein, refers to a biochemical test that measures the presence or concentration of a molecule in a solution through the use of an antibody or an antigen binding fragment thereof that is capable of specifically recognizing said molecule. In a preferred embodiment the immunoassay is an immunoassay based on antibodies bound to Luminex beads, particularly is an immunoassay carried out using Luminex technology, more particularly using a ProcartaPlex^{™} kit.

As previously mentioned, variants of said proteins can be used for measuring the expression levels of MIP-1β, CD62E, IL-8, GM-CSF, IL-1a, MCP-1, ICAM-1, IFN-gamma, IL-1β and IL-12 in order to put into practice the method of the invention.

Thus, variants of the MIP-1β, CD62E, IL-8, GM-CSF, IL-1a, MCP-1, ICAM-1, IFN-gamma, IL-1β and IL-12 proteins may be: (i) one in which one or more of the amino acid residues are substituted with a conserved or non-conserved amino acid residue (preferably a conserved amino acid residue) and such substituted amino acid residue may or may not be one encoded by the genetic code; (ii) one in which there are one or more modified amino acid residues, e.g., residues that are modified by the attachment of substituent groups; (iii) one in which the protein is an isoform or an alternative splice variant of the proteins of the present invention and/or; (iv) fragments of the proteins. The fragments include proteins generated via proteolytic cleavage (including multi-site proteolysis) of an original sequence. Variants are deemed to be within the scope of those skilled in the art from the teaching herein.

Variants according to the present invention include amino acid sequences that are at least 60%, 70%, 80%, 90%, 95% or 96% similar or identical to the original amino acid sequence. As known in the art the "similarity" between two proteins is determined by comparing the amino acid sequence and its conserved amino acid substitutes of one protein to a sequence of a second protein. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be post-translationally modified. For example, post-translational modifications that fall within the scope of the present invention include signal peptide cleavage, glycosylation, acetylation, isoprenylation, proteolysis, myristoylation, protein folding and proteolytic processing, etc. Additionally, the proteins may include unnatural amino acids formed by post-translational modification or by introducing unnatural amino acids during translation.

In a particular embodiment said variant is a mammal variant, preferably a human variant, more preferably with at least 60%, 70%, 80%, 90%, 95% or 96% similarity or identity to the original amino acid sequence.

The second step of the first method of the invention involves comparing the level of said at least one biomarker obtained in step (i) with a reference value.

The term "reference value", as used herein, refers to a laboratory value used as a reference for values/data obtained by means of samples collected from subjects. The reference value or reference level can be an absolute value, a relative value, a value that has an upper or a lower limit, a range of values, an average value, a median value, a mean value, or a value as compared to a particular control or baseline value. A reference value can be based on an individual sample value, such as, for example, a value obtained from a sample from the subject being tested, but at an earlier point in time. The reference value can be based on a large number of samples, for example on a group of patients considered to be representative, such as the values obtained from a population of subjects of a chronological age matched group coinciding with that of the patient object of the study, or based on a pool of samples including or excluding the sample to be tested. Suitable reference values are indicated in the context of the methods of the invention for predicting the response of a subject with cancer that is being treated with an anti-cancer therapy.

In the context of the method of the invention for the prediction of response of a subject suffering from cancer to an anti-cancer treatment, a preferred reference value can be the MIP-1β, CD62E, IL-8, GM-CSF, IL-1a, MCP-1, ICAM-1, IFN-gamma, IL-1β and IL-12 expression level determined in a sample from a subject having cancer or having had cancer which has shown a good clinical response to treatment with the anti-cancer therapy, said expression levels having been determined at the time that the patient was being treated. In another embodiment, the reference value is the MIP-1β, CD62E, IL-8, GM-CSF, IL-1α, MCP-1, ICAM-1, IFN-gamma, IL-1β and IL-12 expression level determined in a sample from a subject having cancer or having had cancer which has shown a poor clinical response to treatment with the anti-cancer therapy, said expression levels having been determined at the time that the patient was being treated. In another embodiment, the reference value is the MIP-1β, CD62E, IL-8, GM-CSF, IL-1a, MCP-1, ICAM-1, IFN-gamma, IL-1β and IL-12 expression level determined in a sample from a pool of subjects having cancer or having had cancer wherein several of them have shown good response and the other have shown bad response to treatment with the anti-cancer therapy, said expression levels having been determined at the time that the patient was being treated. In another embodiment, the reference value is the MIP-1β, CD62E, IL-8, GM-CSF, IL-1a, MCP-1, ICAM-1, IFN-gamma, IL-1β and IL-12 expression level in a healthy patient, i.e., a patient which has not been diagnosed with the type of cancer for which a prediction of the response to therapy is desired.

In an embodiment the reference value can be obtained by determining the median value of expression levels of each of the biomarkers measured in a collection of samples from subjects suffering from cancer who do not have a decrease in said biomarkers or from normal tissue. In an embodiment the reference value can be obtained by determining the median value of expression levels of each of the biomarkers measured in a collection of samples from subjects suffering from cancer who do not have an increase in said biomarkers or from normal tissue.

The collection of samples from which the reference level is derived will preferably be constituted from subjects suffering from the same type of cancer or a mixture of tissues from normal individuals not affected of cancer.

Alternatively, the use of a reference value used for determining whether the expression level of a biomarker is "increased" or "decreased" could correspond to the median value of expression levels of each biomarker measured in a RNA sample obtained by pooling equal amounts of RNA from each of the samples obtained from subjects suffering from cancer who do not have a decrease in the levels of the biomarkers of the invention, preferably from subjects suffering from the same type of cancer. In another embodiment, the reference value can correspond to the median of expression levels of each biomarker measured in a RNA sample obtained by pooling equal amounts of RNA from each of the samples obtained from subjects suffering from cancer who do not have an increase in the levels of the biomarkers of the invention, preferably from subjects suffering from the same type of cancer.

In the present invention, the "reference value" may be an arbitrary cut-off point, established according to ROC methodology. Once this cut-off point is established, the level of this marker expressed in biofluids or tumour tissues from the subject can be compared with this cut-off point, and thus be assigned a level of "low" expression if it is under this cut-off or a level of "high" expression if it is above this cut-off. Therefore, in a preferred embodiment, the expression levels of each of the biomarkers of a combination are compared with predetermined cut off values for each of said genes wherein said predetermined cut off values for each gene preferably correspond to the expression level of said gene which correlate with the highest specificity at said desired sensitivity in a ROC curve calculated based on the expression levels of the biomarkers.

Once this reference value is established, the level of the biomarker expressed in the sample can be compared with said reference value, and thus be assigned a level of "increased", "decreased" or "equal" expression level. For example, an increase in the expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level.

This comparison allows detecting a decrease in the expression of one or more of said biomarkers with respect to the reference value. The expression "decreased levels" of one or more biomarkers selected from the group consisting of MIP-1β, CD62E, IL-8, GM-CSF, IL-1a, MCP-1, ICAM-1, IFN-gamma, IL-1β and IL-12 means a decrease in the expression of a given biomarker with respect to the reference value of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more.

The expression "increased levels" of one or more biomarkers selected from the group consisting of MIP-1β, CD62E, IL-8, GM-CSF IL-1a, MCP-1, ICAM-1, IFN-gamma, IL-1β and IL-12 means an increase in the expression of a given biomarker with respect to the reference value of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more.

Expression levels can be seen as "equal" to the reference value if the levels differ with respect to the reference value less than 5%, less than 4%, less than 3%, less than 2%, less than 1%, less than 0.5 %, less than 0.4%, less than 0.3%, less than 0.1%, less than 0.05%, less than 0.01%, less than 0.001% or less.

When a comparison between the level of one or more biomarkers selected from the group consisting of MIP-1β, CD62E, IL-8, GM-CSF and IL-1α and the reference value has been made, the method of the invention allows making a prediction as to whether the subject will show a good clinical response to a treatment with an anti-cancer treatment according to items (a) to (e) of the first aspect of the invention or a poor clinical response. Thus, subjects having a decreased level of said at least one biomarker with respect to the reference value will have a good clinical response to the anti-cancer treatment.

The expression "good clinical response", as used herein, means that the subject will show a favourable response to the anti-cancer treatment according to items (a) to (e) of the first aspect of the invention, which is understood by the skilled person as a complete response, a partial response or stabilization of the disease.

In a preferred embodiment the good clinical response is stabilization of the disease.

Alternatively, patients having an equal or an increased level of said at least one biomarker with respect to the reference value will have a poor clinical response to the anti-cancer treatment.

The expression "poor clinical response", as used herein, means that the response of the subject is not as favorable as just indicated. Poor clinical response includes a prediction of progression of the disease. It could also include a prediction of relapse, mortality or the necessity to change or administer a new medical treatment.

In a preferred embodiment the poor clinical response is progression of the disease.

As will be understood by those skilled in the art, such the assessment of the probability, although preferred to be, may usually not be correct for 100% of the subjects to be analyzed. The term, however, requires that a statistically significant portion of subjects can be identified as having a predisposition of responding to the chemotherapeutic treatment. Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p- values are, preferably, 0.1, 0.05, 0.01, 0.005, or 0.0001. More preferably, at least 60 percent, at least 70 percent, at least 80 percent or at least 90 percent of the subjects of a population can be properly identified by the method of the present invention.

In a preferred embodiment, the anti-cancer treatment is initiated after predicting the clinical response of a subject suffering from cancer to said treatment by the first method of the invention.

### SELECTIVE METHODS OF THE INVENTION

The authors of the present invention have observed that a decrease in the level of MIP-1β, CD62E, IL-8, GM-CSF or IL-1α with respect to a reference value is indicative of a good clinical response to a therapy with OMO-103. Therefore, the detection of a low level of said biomarkers can be a marker useful for selecting a patient for being treated with a therapy based on Omomyc or a functionally equivalent variant thereof. In this way, subjects can proceed directly to adequate therapies while avoiding less effective therapies or secondary effects associated to them.

Thus, in a second aspect, the invention relates to an *in vitro* method for selecting a customized therapy for a subject suffering from cancer comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value,
wherein:
- a decreased level of said at least one biomarker with respect to the reference value is indicative that the therapy to be selected comprises an agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b); or, alternatively,
- an equal or an increased level of said at least one biomarker with respect to the reference value is indicative that the therapy to be selected does not comprise an agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b).

The term "selecting a customized therapy" or "selecting a personalized therapy", as used herein, refers to selecting appropriate and optimal therapies based on a patient's specific medical characteristics, resulting from its personal background. In the context of the present invention, said characteristic consists of the level of biomarkers in a sample isolated from the patient.

In the context of the second method of the invention the term "therapy" refers to any anti-cancer treatment comprising the exposure of the subject being treated with any method used to induce death of cancerous cells. Said method is not limited to the administration of chemical agents, it can consist on surgery, chemotherapy, radiation therapy, hormonal therapy, targeted therapy including immunotherapy, or a combination thereof.

The term "surgery", when referred to cancer, refers to a major surgery where at least part of the primary tumour and/or at least part of at least one metastasis is/are removed.

The term "chemotherapy", as used herein, is drawn to any treatment consisting in the administration of one or several anti-cancer agents, without chemical structure limitations, suitable for therapy and/or treatment of a cancer, alone or in combination with other compounds.

The term "radiation therapy", "radiotherapy", or "ionizing radiation", as used herein, refers to a therapy using ionizing radiation, generally as part of cancer treatment to control or kill tumor cells and normally delivered by a linear accelerator. Gamma rays, X-rays, and the higher ultraviolet part of the electromagnetic spectrum are ionizing radiation, whereas the lower part of the spectrum below UV, including visible light (including nearly all types of laser light), infrared, microwaves, and radio waves are all considered non-ionizing radiation. Radiation therapy may be curative in a number of types of cancer if they are localized to one area of the body. Radiation therapy is commonly applied to the cancerous tumor because of its ability to control cell growth. Ionizing radiation works by damaging the DNA of cancerous tissue leading to cellular death.

The term "targeted therapy", as used herein, refers to a therapy that blocks the growth of cancer cells by interfering with specific targeted molecules needed for carcinogenesis and tumor growth, rather than by simply interfering with all rapidly dividing cells. Because most agents for targeted therapy are biopharmaceuticals, the term biologic therapy is sometimes synonymous with targeted therapy when used in the context of cancer therapy (and thus distinguished from chemotherapy, that is, cytotoxic therapy). However, the modalities can be combined. Another form of targeted therapy involves the use of nanoengineered enzymes to bind to a tumor cell such that the body's natural cell degradation process can digest the cell, effectively eliminating it from the body. Many targeted therapies are examples of immunotherapy.

The term "immunotherapy", as used herein, refers to cancer immunotherapy consisting in approaches that modify the host immune system, and/or the utilization of components of the immune system as cancer treatment. Non-limiting examples of immunotherapy include the nonspecific immune stimulants BCG and levamisole; the cytokines interferon-a and interleukin-2; the monoclonal antibodies anti-PD1, anti-PDL1, anti-CTLA-4, rituximab, ofatumumab, alemtuzumab, trastuzumab, bevacizumab, cetuximab, and panitumumab; the radiolabeled antibodies Y-90 ibritumomab tiuxetan and 1-131 tositumomab; the immunotoxin denileukin diftitox and the antibody gemtuzumab ozogamicin; nonmyeloablative allogeneic transplants with donor lymphocyte infusions; and the anti-prostate cancer cell-based therapy sipuleucel-T. Other examples of immunotherapies such as DC vaccines, CART-T cell or NK therapy. Some immunotherapies involve the removal of immune cells from the blood or from a tumor and those specific for the tumor are cultured and returned to the patient where they attack the tumor; alternatively, immune cells can be genetically engineered to express a tumor-specific receptor, cultured and returned to the patient. Cell types that can be used in this way are natural killer cells, lymphokine-activated killer cells, cytotoxic T cells and dendritic cells.

The first step of the second method of the invention comprises determining the level of at least one biomarker selected from the group consisting of MIP-1β, CD62E, IL-8, GM-CSF and IL-1α in a sample from said subject; and the second step comprises comparing the level of said at least one biomarker with a reference value.

When a comparison between the level of one or more biomarkers selected from the group consisting of MIP-1β, CD62E, IL-8, GM-CSF and IL-1α and the reference value has been made, the method of the invention allows deciding the suitable treatment for the subject tested. Thus, subjects having a decreased level of said at least one biomarker with respect to the reference value should select a therapy comprising an agent selected from the group consisting of items (a) to (e) of the first aspect of the invention.

As used herein, an "anti-cancer agent" is an agent that at least partially inhibits the development or progression of a cancer, or that stabilizes the disease, including inhibiting in whole or in part symptoms associated with the cancer even if only for the short term.

On the other hand, subjects having an equal or an increased level of said at least one biomarker with respect to the reference value should select an alternative therapy, i.e., a therapy that does not comprise an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b).

The expression "alternative therapy", in the context of the second method of the invention, refers to any therapy different from a therapy comprising an agent defined in items (a) to (e) of the first method of the invention. Alternative therapy, includes, without limitation, surgery, chemotherapy, radiation therapy, hormonal therapy and targeted therapy, including immunotherapy, and combinations thereof.

Illustrative, non-limitative examples of alternative therapies include, surgery, treatments with anti-cancer agents such as chemotherapy agents including anthracycline antibiotics such as doxorubicin and daunorubicin, taxanes such as Taxol^{™} and docetaxel, vinca alkaloids such as vincristine and vinblastine, 5-fluorouracil (5-FU), leucovorin, irinotecan, idarubicin, mitomycin C, oxaliplatin, raltitrexed, tamoxifen, cisplatin, carboplatin, methotrexate, actinomycin D, mitoxantrone, blenoxane or mithramycin; toxins such as the ricin A-chain, saponin, the diphtheria A-chain, active non-binding fragments of the diphtheria toxin, *Pseudomonas aeruginosa* exotoxin A-chain, abrin A-chain, modecin A-chain, α-sarcin, *Leurites fordii* A-proteins, dianthin proteins, *Phytolaca americana* (PAPI, PAPII and PAP-S) proteins, *Momordica charantia* inhibitor, curcine, crotin, *Saponaria officinalis* inhibitor, gelonin, mitogelin, restrictocin, phenomycin, enomycin and trichothecenes; enzymes such as alkaline phosphatase which activates etoposide and doxorubicin; carboxypeptidase G2 which activates nitrogen mustards; beta-lactamase which activates doxorubicin, paclitaxel and mitomycin; cytokines such as TNF factor alpha, INF-gamma, GM-GSF factor or IL-2; radioisotopes such as ¹³¹I, ⁹⁰Y, ¹⁷⁷Lu, ¹⁸⁸Re, ⁶⁷Cu, ²¹¹At, ²¹³Bi, ¹²⁵I, ¹¹¹In; antiangiogenic agents such as paclitaxel, 2-methoxyestradiol, prinomastat, batimastat, BAY 12-9566, carboxyamidotriazole, CC-1088, dextromethorphan acetic acid, dimethylxanthenone acetic acid, endostatin, IM-862, marimastat, penicillamine, PTK787/ZK 222584, RPI.4610, squalamine lactate, SU5416, thalidomide, combretastatin, tamoxifen, COL-3, neovastat, BMS-275291, SU6668, anti-VEGF antibodies, Medi-522 (Vitaxin II), CAI, interleukin 12, IM862, amiloride, angiostatin, KI-3 angiostatin, KI-5 angiostatin, Captopril, DL-alpha-difluoromethylornithine, DL-alpha-difluoromethylornithine HCI, endostatin, fumagillin, herbimycin A, 4-Hydroxyphenylretinamide, juglone, laminin, laminin hexapeptide, laminin pentapeptide, lavendustin A, medroxyprogesterone, minocycline, placenta ribonuclease inhibitor, suramin, thrombospondin, antibodies directed against proangiogenic factors (for example, Avastin, Erbitux, Vectibix, Herceptin); low molecular weight tyrosine kinase inhibitors of proangiogenic growth factors (for example Tarceva, Nexavar, Sutent, Iressa); mTOR inhibitors (for example Torisel); interferon alpha, beta and gamma, IL-12, matrix metalloproteinase inhibitors (for example, COL3, marimastat, batimastat); ZD6474, SUI1248, vitaxin; PDGFR inhibitors (for example Gleevec); NM3 and 2- ME2; cyclopeptides such as cilengitide; antiproliferative agents such as (i) antimetabolites such as folic acid antimetabolites (aminopterin, denopterin, methotrexate, edatrexate, trimetrexate, nolatrexed, lometrexol, pemetrexed, raltitrexed, piritrexim, pteropterin, leucovorin, 10-propargyl-5,8-dideazafolate (PDDF, CB3717)), purine analogs (cladribine, clofarabine, fludarabine, mercaptopurine, pentostatin, thioguanine) and pyrimidine analogs (capecitabine, cytarabine or ara-C, decitabine, fluorouracil, 5-fluorouracil, doxifluridine, floxuridine and gemcitabine) (ii) natural products, such as antitumor antibiotics and mitotic inhibitors such vinca alkaloids such as vindesine, vincristine, vinblastine, vinorelbine; taxanes such as paclitaxel (Taxol^{™}), docetaxel (Taxotere^{™}); colchicine (NSC 757), thiocolchicine (NSC 361792), colchicine derivatives (e. g., NSC 33410), and allocolchicine (NSC 406042); halichondrin B (NSC 609395); dolastatin 10 (NSC 376128); maytansine (NSC 153858); rhizoxin (NSC 332598); epothilone A, epothilone B; discodermolide; estramustine; nocodazole; (iii) hormones and antagonist thereof, such tamoxifen, toremifene, anastrozole, arzoxifene, lasofoxifene, raloxifene, nafoxidine, fulvestrant, aminoglutethimide, testolactone, atamestane, exemestane, fadrozole, formestane, letrozole, goserelin, leuprorelin or leuprolide, buserelin, histrelin, megestrol and fluoxymesterone; (iv) biological agents, such as viral vectors, interferon alpha and interleukines; (v) platinum based compounds such as carboplatin, cisplatin [cis-diamminedichloroplatinum, (CDDP)], oxaliplatin, iproplatin, nedaplatin, triplatin tetranitrate, tetraplatin, satraplatin (JM216), JM118 [cis ammine dichloro (II)], JM149 [cis ammine dichloro (cyclohexylamine) trans dihydroxo platinum (IV)], JM335 [trans ammine dichloro dihydroxo platinum (IV)], transplatin, ZD0473, cis, trans, cis-Pt(NH3)(C6H11NH2)(OOCC3H7)2Cl, malanate-I,2-diaminociclohexanoplatin(II), 5-sulphosalycilate-trans-(I,2- diaminociclohexane)platin (II) (SSP), poly-[(trans-I,2-diaminocyclohexane)platin]- carboxyamilose (POLY-PLAT) and 4-hydroxy-sulphonylphenylacetate (trans- 1,2- diaminocyclohexane) platinum (II) (SAP) and the like and (vi) DNA-alkylating drugs such as nitrogen mustards, nitrosoureas, ethylenimine derivatives, alkyl sulfonates and triazenes, including, but not limited to, cyclophosphamide (Cytoxan^{™}), busulfan, improsulfan, piposulfan, pipobroman, melphalan (L-sarcolysin), chlorambucil, mechlorethamine or mustine, uramustine or uracil mustard, novembichin, phenesterine, trofosfamide, ifosfamide, carmustine (BCNU), lomustine (CCNU), chlorozotocin, fotemustine, nimustine, ranimnustine, semustine (methyl-CCNU), streptozocin, thiotepa, triethylenemelamine, triethylenethiophosphoramine, procarbazine, altretamine, dacarbazine, mitozolomide and temozolomide; targeted therapy or immunotherapy such as anti-PD1 or anti-CTLA4.

In a third aspect, the invention relates to an *in vitro* method for selecting a subject suffering from cancer for a treatment comprising an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
said method comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value, wherein the patient is selected for said treatment if a decreased level of said at least one biomarker with respect to the reference value is detected.

The first step of the third method of the invention comprises determining the level of at least one biomarker selected from the group consisting of MIP-1β, CD62E, IL-8, GM-CSF and IL-1α in a sample from said subject; and the second step comprises comparing the level of said at least one biomarker with a reference value.

When a comparison between the level of one or more biomarkers selected from the group consisting of MIP-1β, CD62E, IL-8, GM-CSF and IL-1α and the reference value has been made, the method of the invention allows selecting a patient to undergo a treatment. Particularly, the subjects having a decreased level of said at least one biomarker with respect to the reference value are selected for a treatment with any of the items (a) to (e) of the first aspect of the invention.

Any treatment and pharmaceutical form of the treatment of the invention can be used as defined in the context of the fourth aspect of the invention.

The skilled person will appreciate that the particular embodiments developed in the first aspect of the invention are also applicable to the second and third aspects of the invention.

All the terms have been described in detail above in the context of the first aspect of the invention and are used with the same meaning in the context of the second and third aspects of the invention.

### THERAPEUTIC METHODS OF THE INVENTION

The results obtained in the present invention show that a low level of MIP-1β, CD62E, IL-8, GM-CSF and IL-1α in a sample of a patient suffering from cancer taken before the treatment has started, indicates a high probability of a successful therapeutic treatment with an anti-cancer treatment as defined in items (a) to (f) of the first aspect of the invention. This is an indication that this may be the best treatment available for such patients. An anti-cancer treatment with items (a) to (f) of the first aspect of the invention would be the choice as first line treatment for patients with low levels of MIP-1β, CD62E, IL-8, GM-CSF and IL-1α.

In a fourth aspect, the invention relates to an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
for use in the treatment of cancer in a subject, wherein the subject has been identified as a good responder to said agent by a method according to the first aspect of the invention, or wherein the treatment has been selected by the second method of the invention.

Alternatively, the invention relates to the use of an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
for the manufacture of a medicament for the treatment of a subject suffering from cancer, wherein the subject has been identified as a good responder to said agent by a method according to the first aspect of the invention, or wherein the treatment has been selected by the second method of the invention.

Alternatively, the invention relates to a method for the treatment of cancer in a subject comprising administering to said subject an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
wherein the subject has been identified as a good responder to said agent by a method according to the first aspect of the invention or wherein the treatment has been selected by the second method of the invention.

The agent preferably forms part of a medicament, i.e., a composition comprising a therapeutically effective amount of the agent comprising at least one pharmaceutically acceptable excipient or carrier. The medicament can comprise more than one agent, either more than one agent defined under items (a) to (e) of the first aspect of the invention or additional agents useful for the treatment of cancer.

The term "therapeutically effective amount", as used herein, refers to the sufficient amount of the agent to provide the desired effect and will generally be determined by, among other causes, the characteristics of the compound itself and the therapeutic effect to be achieved. It will also depend on the subject to be treated, the severity of the cancer disease suffered by said subject, the chosen dosage form, administration route, etc.

The expression "pharmaceutically acceptable excipient" or "pharmaceutically acceptable carrier", as used herein, refers to any compound or combination of compounds that is essentially non-toxic to the subject at the dosage and concentration employed, and is compatible with the other components of a medicament. Thus, an excipient is an inactive substance formulated alongside the active ingredient of a medicament for the purpose of bulking-up compositions that contain said active ingredients. Excipients also can serve various therapeutic enhancing purposes, such as facilitating the active ingredient absorption or solubility, or other pharmacokinetic considerations. Excipients can also be useful in the manufacturing process, to aid in the handling of the active substance concerned such as by facilitating powder flowability or non-stick properties, in addition to aiding *in vitro* stability such as prevention of denaturation over the expected shelf life.

In a preferred embodiment the anticancer treatment is administered intravenously; preferably as 30 minutes intravenous infusion. In an embodiment, the anti-cancer therapy is administered once a week.

In a preferred embodiment the agent is a polypeptide comprising the sequence SEQ ID NO: 1 or a functionally equivalent variant thereof; more preferably is a polypeptide consisting of SEQ ID NO: 4.

All the embodiments of the first, second and third aspects of the invention are also applicable to the fourth aspect of the invention.

All the terms have been defined previously in the context of the first, second and third aspects of the invention and are used with the same meaning in the context of the fourth aspect of the invention.

### KITS OF THE INVENTION AND USES THEREOF

The inventors of the present invention have found that the levels of MIP-1β, CD62E, IL-8, GM-CSF and IL-1α can be determined by an immunoassay to predict the response of a subject suffering from cancer to an anti-cancer treatment with OMO-103.

In another aspect, the invention relates to a kit comprising a reagent specific for determining the expression level of at least one biomarker selected from the group consisting of MIP-1β, CD62E, IL-8, GM-CSF and IL-1α.

In another aspect, the invention relates to the use of a kit comprising a reagent specific for determining the expression level of at least one biomarker selected from the group consisting of MIP-1β, CD62E, IL-8, GM-CSF and IL-1α, or to the use of a reagent specific for determining the expression level of at least one biomarker selected from the group consisting of MIP-1β, CD62E, IL-8, GM-CSF, IL-1α, MCP-1, ICAM-1, IFN-gamma, IL-1β, and IL-12, preferably selected from the group consisting of MIP-1β, CD62E, IL-8, GM-CSF, IL-1α, in a sample from a subject suffering from cancer for predicting the clinical response of said subject to a treatment with any of the agents defined in items (a) to (e) of the first aspect of the invention. In a preferred embodiment the kit or reagent is used in a method according to the first aspect of the invention.

In another aspect, the invention relates to the use of a kit comprising a reagent specific for determining the expression level of at least one biomarker selected from the group consisting of MIP-1β, CD62E, IL-8, GM-CSF and IL-1α, or to the use of a reagent specific for determining the expression level of at least one biomarker selected from the group consisting of MIP-1β, CD62E, IL-8, GM-CSF, IL-1α, MCP-1, ICAM-1, IFN-gamma, IL-1β, and IL-12, preferably selected from the group consisting of MIP-1β, CD62E, IL-8, GM-CSF and IL-1α, in a sample from a subject suffering from cancer for selecting a therapy for said subject, particularly a therapy with any of the agents defined in items (a) to (e) of the first aspect of the invention. In a preferred embodiment the kit or reagent is used in a method according to the second aspect of the invention.

In another aspect, the invention relates to the use of a kit comprising a reagent specific for determining the expression level of at least one biomarker selected from the group consisting of MIP-1β, CD62E, IL-8, GM-CSF and IL-1α, or to the use of a reagent specific for determining the expression level of at least one biomarker selected from the group consisting of MIP-1β, CD62E, IL-8, GM-CSF, IL-1α, MCP-1, ICAM-1, IFN-gamma, IL-1β, and IL-12, preferably selected from the group consisting of MIP-1β, CD62E, IL-8, GM-CSF and IL-1α, in a sample from a subject suffering from cancer for selecting a cancer patient for being treated with any of the agents defined in items (a) to (e) of the first aspect of the invention. In a preferred embodiment the kit or reagent is used in a method according to the third aspect of the invention.

In the context of the present invention, "kit" is understood as a product containing the different reagents necessary for carrying out the different uses of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits used in the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials.

Additionally or alternatively, the media can contain internet addresses that provide said instruction.

In a preferred embodiment the kit further comprises reagents specific for determining the expression level of at least one additional biomarker selected from the group consisting of MCP-1, ICAM-1, IFN-gamma, IL-1β and IL-12 combinations thereof.

In a more preferred embodiment, the kit comprises a reagent specific for determining the expression level of MIP-1β and at least an additional reagent specific for determining the expression level of a biomarker selected from the group consisting of CD62E, IL-8, GM-CSF, IL-1α, MCP-1, ICAM-1, IFN-gamma, IL-1β and IL-12.

The term "reagent", as used herein, refers to any compound or composition which can be used for detecting the level of any of the biomarkers of the present invention, for example the level of any of the biomarkers MIP-1β, CD62E, IL-8, GM-CSF and IL-1α or the level of any of the biomarkers MCP-1, ICAM-1, IFN-gamma, IL-1β and IL-12. In a preferred embodiment the reagent is a reagent for detecting the level of MIP-1β. In another embodiment the reagent is a reagent for detecting the level of CD62E. In another embodiment the reagent is a reagent for detecting the level of IL-8. In another embodiment the reagent is a reagent for detecting the level of GM-CSF. In another embodiment the reagent is a reagent for detecting the level of IL-1α. In another embodiment the reagent is a reagent for detecting the levels of one or more biomarkers selected from the group consisting of MIP-1β, CD62E, IL-8, GM-CSF, IL-1α, MCP-1, ICAM-1, IFN-gamma, IL-1β and IL-12, i.e., for detecting their genes, proteins or variants thereof. The reagent can include, optionally, reagents for detecting one or more housekeeping genes or the protein encoded by said housekeeping gene(s).

The expression "reagent specific for determining the expression level" of a biomarker, as used herein, refers to any compound or set of compounds that allows the specific determination of the expression level of a gene or protein by detection methods well known by the person skilled in the art. Particularly, the term "specific reagent", as used herein, refers to any reagent that can be used for the specific quantification of one of the biomarkers of the invention.

In an embodiment, the reagent specific for determining the expression level of a biomarker is a nucleic acid capable of specifically hybridizing with any of the MIP-1β, CD62E, IL-8, GM-CSF, IL-1α, MCP-1, ICAM-1, IFN-gamma, IL-1β and IL-12 genes. In a preferred embodiment said nucleic acid is an oligonucleotide that hybridize specifically to the biomarker.

The term "oligonucleotide", as used herein, refers to a nucleic acid having preferably at least 10 nucleotides, preferably at least 15 nucleotides, preferably at least 20 nucleotides, preferably at least 25 nucleotides and preferably no more than 100 nucleotides, including polyribonucleotides, polydeoxyribonucleotides and combinations thereof. The term "oligonucleotide" also refers to molecules formed by conventional nucleotides bound by phosphodiester conventional bonds as well as variants thereof including modifications in purines or pyrimidines or modifications in riboses or deoxyriboses designed to increase the stability of the oligonucleotide. Alternatively or additionally, the oligonucleotides may contain modified bonds such as phosphotriester, phosphorothioate, methylphosphonate, etc, or may be peptide nucleic acids (PNA). Methods to synthesize oligonucleotides are well known by the person skilled in the art. The oligonucleotide can be a primer or a probe. In an embodiment the oligonucleotide is a probe. In another embodiment the oligonucleotide is a primer, preferably a pair of primers. In an embodiment, the present invention provides a set of one or more pairs of oligonucleotide primers designed to specifically amplify the biomarker of the methods of the invention.

Nucleic acids capable of specifically hybridizing with the MIP-1β, CD62E, IL-8, GM-CSF, IL-1α, MCP-1, ICAM-1, IFN-gamma, IL-1β and IL-12 genes are, for example, one or more pairs of primer oligonucleotides for the specific amplification of fragments of the mRNA (or of their corresponding cDNA) of said gene.

As the skilled person understands, the oligonucleotide primers and probes of the kit of the invention can be used in all techniques of gene expression profiling (RT-PCR, SAGE, TaqMan, Real Time-PCR, FISH, NASBA, etc.).

In another embodiment, the reagent specific for determining the expression level of a biomarker is a compound that specifically binds to the biomarker protein, particularly is selected from the group consisting of antibodies, aptamers and fragments thereof.

In a preferred embodiment, the reagent specific for determining the expression level of a biomarker is an antibody or fragment thereof capable of specifically binding to (i.e., specifically recognizing) MIP-1β, CD62E, IL-8, GM-CSF, IL-1α, MCP-1, ICAM-1, IFN-gamma, IL-1β or IL-12 proteins or to variants thereof (including fragments thereof containing antigenic determinants).

Antibodies, or a fragment thereof, capable of detecting an antigen, capable of specifically binding to a protein or to variants thereof are, for example, monoclonal and polyclonal antibodies, antibody fragments, Fv, Fab, Fab' and F(ab')2, ScFv, diabodies, triabodies, tetrabodies and humanised antibodies. The antibodies of the kit of the invention can be used in conventional methods for detecting protein expression levels, such as Western-blot or Western transfer, ELISA (enzyme linked immunosorbent assay), RIA (radioimmunoassay), competitive EIA (enzymatic immunoassay), DAS-ELISA (double antibody sandwich ELISA), immunocytochemical and immunohistochemical techniques, techniques based on the use of biochips, protein microarrays including specific antibodies or assays based on colloidal precipitation in formats such as dipsticks, etc. In a preferred embodiment said antibodies are used in an immunoassay. Preferably, the immunoassay is based on luminex technology, more preferably is a ProcartaPlex^{™} kit.

Said reagents, specifically the probes and the antibodies, may be fixed onto a solid support, such as a membrane, a plastic or a glass, optionally treated in order to facilitate fixation of said probes or antibodies onto the support.

The kits of the invention optionally comprise additional reagents for detecting a housekeeping gene, a polypeptide encoded by a housekeeping gene or the mRNA encoded by said housekeeping gene. The availability of said additional reagent allows the normalization of measurements taken in different samples (e.g. the test sample and the control sample) to exclude that the differences in expression of the biomarker are due to a different amount of total protein in the sample rather than to real differences in relative expression levels. Housekeeping genes, as used herein, relates to genes which code for proteins which are constitutively expressed and carry out essential cellular functions. Preferred housekeeping genes for use in the present invention include β-2-microglobulin, ubiquitin, 18-S ribosomal protein, cyclophilin, PSMB4, GAPDH, tubulin and β-actin.

In a preferred embodiment, the kit referred in any of the uses above, further comprises a reagent specific for detecting or determining the expression level of a gene or protein whose expression determines whether a cell in a sample is proliferating. Preferably, the kit comprises an antibody capable of specifically recognizing a protein whose expression determines whether a cell in a sample is proliferating, preferably selected from the group consisting of geminin, KI-67, proliferating cell nuclear antigen, cyclin A2.

The kits of the invention also contain other reagents that allow determining the expression level of a biomarker but that are not specific for said biomarker, e.g., reagents for the extraction of RNA material, etc., e.g., primers for the synthesis of the corresponding cDNA by means of RT, reagents for the amplification of DNA such as DNA polymerases, dNTPs, buffers, etc.

In another preferred embodiment, any of the reagents referred in this section, particularly any of the oligonucleotides or antibodies referred in this section, more preferably any of the antibodies comprise each one at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, or at least 100% of the total amount of reagents forming the kits of the invention, preferably of the total amount of reagents specific for the determination of the level of expression of one or more biomarkers forming the kit.

In a more preferred embodiment, the reagent specific for determining the expression level of at least one biomarker comprises at least 10%, at least 20%, at least 30%, at least 40%, at least 50% of the total amount of reagents forming the kit. Preferably, at least 10%, at least 20%, at least 30%, at least 40%, at least 50% of the reagents specific for determining biomarkers comprised in the kit are reagents specific for determining the expression level of at least one biomarker selected from the group consisting of MIP-1β, CD62E, IL-8, GM-CSF, IL-1α, MCP-1, ICAM-1, IFN-gamma, IL-1β and IL-12.

All the particular embodiments disclosed for the methods of the present invention are applicable to the kit of the invention and uses thereof.

All the terms have been defined previously in the context of the first, second, third and fourth aspects of the invention and are used with the same meaning in the context of the kits of the invention and uses thereof.

The invention also relates to the following aspects:
1. An *in vitro* method for predicting the clinical response of a subject suffering from cancer to an anti-cancer treatment selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b);
   said method comprising:
   (i) determining the level of at least one biomarker selected from the group consisting of MIP-1β, CD62E, IL-8, GM-CSF and IL-1a in a sample from said subject; and
   (ii) comparing the level of said at least one biomarker with a reference value,
   wherein:
   - a decreased level of said at least one biomarker with respect to the reference value is indicative of a good clinical response of the subject to the anti-cancer treatment, or
   - an equal or an increased level of said at least one biomarker with respect to the reference value is indicative of a poor clinical response of the subject to the anti-cancer treatment.
2. The method according to aspect 1, wherein the good clinical response is stabilization of the disease.
3. The method according to any one of aspects 1 or 2, wherein the poor clinical response is progression of the disease.
4. An *in vitro* method for selecting a customized therapy for a subject suffering from cancer comprising:
   (i) determining the level of at least one biomarker selected from the group consisting of MIP-1β, CD62E, IL-8, GM-CSF and IL-1α in a sample from said subject; and
   (ii) comparing the level of said at least one biomarker with a reference value,
   wherein:
   - a decreased level of said at least one biomarker with respect to the reference value is indicative that the therapy to be selected comprises an agent selected from the group consisting of:
      a) a polypeptide comprising the sequence SEQ ID NO: 1 or a functionally equivalent variant thereof;
      b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
      c) a polynucleotide encoding the polypeptide of a) or the conjugate of b);
      d) a vector comprising the polynucleotide according to c); and
      e) a cell capable of secreting into the medium the polypeptide according to a) or the conjugate according to b); or, alternatively,
   - an equal or an increased level of said at least one biomarker with respect to the reference value is indicative that the therapy to be selected does not comprise an agent selected from the group consisting of:
      a) a polypeptide comprising the sequence SEQ ID NO: 1 or a functionally equivalent variant thereof;
      b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
      c) a polynucleotide encoding the polypeptide of a) or the conjugate of b);
      d) a vector comprising the polynucleotide according to c); and
      e) a cell capable of secreting into the medium the polypeptide according to a) or the conjugate according to b).
5. An *in vitro* method for selecting a subject suffering from cancer for a treatment comprising an agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b);
   said method comprising:
   (i) determining the level of at least one biomarker selected from the group consisting of MIP-1β, CD62E, IL-8, GM-CSF and IL-1α in a sample from said subject; and
   (ii) comparing the level of said at least one biomarker with a reference value, wherein the patient is selected for said treatment if a decreased level of said at least one biomarker with respect to the reference value is detected.
6. The method according to any one of aspects 1 to 5, wherein the subject has not received any anti-cancer treatment within the last 24 hours prior to the isolation of the sample.
7. The method according to aspect 6, wherein the subject has not received systemic anti-cancer treatment within at least 4 weeks prior to the isolation of the sample.
8. The method according to any one of aspects 1 to 7, wherein the sample is blood, serum or plasma.
9. The method according to aspect 8, wherein the sample is a sample of serum.
10. The method according to any one of aspects 1 to 9, wherein the levels of at least one biomarker that are determined are mRNA levels.
11. The method according to any one of aspects 1 to 9, wherein the levels of at least one biomarker that are determined are protein levels.
12. The method according to aspect 11, wherein the level of at least one biomarker protein is determined by an immunoassay.
13. The method according to any one of aspects 1 to 12, wherein the anti-cancer treatment comprises the use of a polypeptide comprising the sequence SEQ ID NO: 1 or a functionally equivalent variant thereof.
14. The method according to aspect 13, wherein the anti-cancer treatment comprises the use of a polypeptide consisting of SEQ ID NO: 4.
15. The method according to any one of aspects 1 to 14, wherein the cancer is a solid tumor.
16. The method according to any one of aspects 1 to 15, wherein the subject from whom the sample has been isolated hast not received any anti-cancer treatment before the sample is isolated.
17. The method according to any one of aspects 1 to 15, wherein the subject from whom the sample has been isolated has received an anti-cancer treatment before the isolation of the sample, and wherein the anti-cancer treatment is different from a treatment selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b).
18. The method according to any one of aspects 1 to 17, further comprising determining the level of at least one additional biomarker selected from the group consisting of MCP-1, ICAM-1, IFN-gamma, IL-1β, IL-12 and combinations thereof.
19. The method according to any one of aspects 1 to 18, wherein the method comprises determining the levels of a combination of biomarkers selected from the group consisting of:
   a) a combination of biomarkers comprising MIP-1β and MCP-1;
   b) a combination of biomarkers comprising MIP-1β and ICAM-1;
   c) a combination of biomarkers comprising MIP-1β and CD62E;
   d) a combination of biomarkers comprising MIP-1β and IFN-gamma;
   e) a combination of biomarkers comprising MIP-1β and IL-1β;
   f) a combination of biomarkers comprising MIP-1β and IL-12; and
   g) a combination of biomarkers comprising CD62E and IL-8.
20. The method according to aspect 19, wherein the method comprises determining the levels of a combination of biomarkers selected from the group consisting of:
   a) a combination of biomarkers comprising MIP-1β and MCP-1; and
   b) a combination of biomarkers comprising CD62E and IL-8.
21. The method according to any one of aspects 19 or 20, wherein the method comprises determining the levels of MIP-1β, CD62E, IL-8, GM-CSF and IL-1α.
22. An agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b);
   for use in the treatment of cancer in a subject, wherein the subject has been identified as a good responder to said agent by a method as defined in any one of aspects 1 to 3 or 6 to 21.
23. An agent selected from the group consisting of:
   a) a polypeptide comprising the sequence SEQ ID NO: 1 or a functionally equivalent variant thereof;
   b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
   c) a polynucleotide encoding the polypeptide of a) or the conjugate of b);
   d) a vector comprising the polynucleotide according to c); and
   e) a cell capable of secreting into the medium the polypeptide according to a) or the conjugate according to b);
   for use in the treatment of cancer in a subject, wherein the treatment has been selected by a method as defined in any one of aspects 4 or 6 to 21.
24. The agent for use according to any one of aspects 22 or 23, wherein the agent is a polypeptide comprising the sequence SEQ ID NO: 1 or a functionally equivalent variant thereof.
25. The agent for use according to aspect 24, wherein the agent is a polypeptide consisting of SEQ ID NO: 4.
26. A kit comprising a reagent specific for determining the expression level of at least one biomarker selected from the group consisting of MIP-1β, CD62E, IL-8, GM-CSF and IL-1α.
27. The kit according to aspect 26, further comprising reagents specific for determining the expression level of at least one additional biomarker selected from the group consisting of MCP-1, ICAM-1, IFN-gamma, IL-1β, IL-12 and combinations thereof.
28. The kit according to aspect 27, wherein the kit comprises a reagent specific for determining the expression level of MIP-1β and at least an additional reagent specific for determining the expression level of a biomarker selected from the group consisting of CD62E, IL-8, GM-CSF, IL-1α, MCP-1, ICAM-1, IFN-gamma, IL-1β, and IL-12.
29. The kit according to any one of aspects 26 to 28, wherein the reagent is an antibody capable of specifically recognizing at least one of the biomarkers.
30. The kit according to any one of aspects 26 to 29, wherein the reagent specific for determining the expression level of at least one biomarker comprises at least 10% of the total amount of reagents forming the kit.
31. Use of a kit according to any one of aspects 26 to 30 or of a reagent specific for determining the expression level of at least one biomarker selected from the group consisting of MIP-1β, CD62E, IL-8, GM-CSF IL-1α, MCP-1, ICAM-1, IFN-gamma, IL-1β, and IL-12 in the method as defined in any one of aspects 1 to 21.

The invention will be described by way of the following examples which are to be considered as merely illustrative and not limitative of the scope of the invention.

### EXAMPLES

### Experimental details

The product administered was OMO-103 (SEQ ID NO: 4), which corresponds to the Omomyc peptide sequence further including a methionine at the N-terminal end. OMO-103 is the final drug product, consisting of the active pharmaceutical ingredient (API) and a mixture of excipients, to be used in the clinic for intravenous infusion.

Patients had not received systemic anti-cancer treatment within at least 4 weeks prior to the isolation of the sample, and they had not received any anti-cancer treatment at least within 24 hours prior to the isolation of the sample.

The patients had different kind of solid tumors, including pancreatic ductal adenocarcinoma (PDAC), non-small cell lung cancer (NSCLC), colorectal cancer (CRC), salivary gland carcinoma, sarcoma, triple negative breast cancer and pleural mesothelioma.

Blood samples from the patients included in the Phase I of the OMO-103 clinical trial were taken at pretreatment, just before the patients received the first OMO-103 infusion. OMO-103 was administered as 30 minutes intravenous infusion at dosis 2.88 mg/kg, 4.32 mg/kg, 6.48 mg/kg and 9.72 mg/kg. Then, the serum was isolated using standard procedures and cryopreserved at -80°C until its use. Cytokine and chemokines levels were measured by the Luminex technique using the Inflammation 20-Plex Human ProcartaPlex^{™} kit (Invitrogen). Cytokine and chemokine levels were extrapolated from the standard curves using the ProcartaPlex Analyst Software.

Patient response to OMO-103 was measured after three treatment cycles (9 weeks) through CT scans using the RECIST 1.1 criteria (Eisenhauer, E.A., et al. (2009). New response evaluation criteria in solid tumors: revised RECIST guideline (version 1.1), Eur J Cancer 45: 228-247). Depending on the RECIST results, patients were then classified between Progressive Disease (PD) and Stable Disease (SD). In total, 18 patients were evaluable for the cytokine and chemokine analysis, 11 were classified as PD and 7 as SD.

In order to determine if the levels of the different cytokines and chemokines were different between PD and SD patients at pretreatment, a univariate analysis was performed. A Mann-Whitney U test was used to determine if the levels of the cytokines/chemokines between the two groups were significantly different (Figure 1).

The diagnostic accuracy of individual cytokines and chemokines to identify SD patients by using the area under the receiver operator characteristic curve (ROC-AUC) was estimated (Figure 2). As a global measure to compare the predictive power of each individual cytokine/chemokine, the ROC-AUC was used. In general, an AUC of 0.5 suggests no discrimination, 0.7 to 0.8 is considered acceptable, 0.8 to 0.9 is considered excellent, and more than 0.9 is considered outstanding, knowing that a model is a perfect classifier when the AUC is 1 (DW Hosmer, S Lemeshow (2000). Applied Logistic Regression, 2nd Ed. Chapter 5, John Wiley and Sons, New York, NY (2000), pp. 160-164). Analysis was performed using fitting a logistic regression model to each cytokine individually, with RECIST (C3) as the binary output variable. The Python package Scikit-learn (Pedragosa F. et al. (2011). Scikit-learn: Machine Learning in Python. Journal of Machine Learning Research, 12, pp. 2825-2830) was used to fit the models.

Combinations of cytokines/chemokines that can correctly stratify patients between PD and SD were found using the QLattice modelling technology (Abzu) (René, K et al. (2021). An approach to symbolic regression using Feyn. arXiv:2014.05417 [cs.LG]; Wilsup, C. et al. (2021). Symbolic regression outperforms other models for small datasets. arXiv:2013.15147 [cs.LG]; Christensen, N. et al. (2022) Identifying interactions in omics data for clinical biomarker discovery using symbolic regression, Bioinformatics, Volume 38, Issue 15: 3749-3758). The QLattice is a symbolic regression algorithm that searches for the combinations of input variables and mathematical functions that provide prediction to the output variable. It employs an evolutionary approach to conduct the search and selects the best models by minimizing an error metric calculated between prediction and true output. QLattice was ran within a Leave-one-out cross-validation loop, i.e., the algorithm was ran 16 times, excluding a single patient at each iteration. There were two models that appeared in each iteration: MCP-1 + MIP-1β and CD62E + IL-8. CD62E + MIP-1β and ICAM-1 + MIP-1β appeared 15 times; IFN-gamma + MIP-1β and IL-1β + MIP-1β appeared 14 times; and IL-12 + MIP-1β appeared 13 times. ROC-AUC was also calculated for all the combination models to determine its prediction accuracy and power (Figure 3). The confidence interval bands of the models were estimated using the parameter values of all 16 models found.

### Results

Patients that responded to OMO-103 treatment presenting a stabilization of the disease at cycle 3 displayed significantly low levels of CD62E, IL-8, MIP-1β, GM-CSF and IL-1α at baseline compared to the levels shown by patients who displayed progressive disease (Figure 1).

In order to investigate the predictive power of the identified cytokines and chemokines to the response to OMO-103, receiver operating characteristic (ROC) curve analysis was performed (Figure 2). The ROC-AUC of the five significant decreased cytokines/chemokines were 0.87 for CD62E, 0.91 for IL-8, 0.97 for MIP-1β, 0.82 for GM-CSF and 0.79 for IL-1 α. All values were close to 0.8 or above, which indicate that they represent excellent SD outcome predictors and have a very potent prognostic power.

Finally, QLattice modelling technology was used in order to find combinations of cytokines that could stratify and distinguish which patients would respond to the therapy at baseline showing stabilization of the disease. This analysis revealed that different cytokine/chemokine combinations could correctly classify SD and PD patients: CD62E + IL-8, MIP-1β and MCP-1, MIP-1β + ICAM-1, MIP-1β + CD62E, MIP-1β + IFN-gamma, MIP-1β + IL-1β and MIP-1β + IL-12 (Figure 3A).

For the CD62E + IL-8 combination model, when IL-8 > 8.0 pg/ml, patients have a probability of developing PD above 80% independently of CD62E and, when CD62E > 33,880 pg/ml, patients have a probability of developing PD above 80% independently of IL-8. On the other hand, for IL-8 below 8.0 pg/ml, as CD62E increases, the critical threshold value of IL-8 decreases, while the opposite happens for CD62E below 33,880 pg/ml. For the combination of MIP-1β and MCP-1, when MIP-1β > 65 pg/ml, patients have a probability of developing PD of 80% independently of MCP-1 and, when MIP-1β is below 65 pg/ml, as MCP-1 increases, the critical threshold value of MIP-1β decreases.

For the ICAM-1 + MIP-1β model, when MIP-1β > 55.96 pg/ml, patients have a probability of developing PD above 80% independently of ICAM-1 levels. On the other hand, when MIP-1β < 55.96 pg/ml, this critical threshold decreases as the levels of ICAM-1 increase. For the CD62E + MIP-1β model, when MIP-1β > 117.06 pg/ml, patients have a probability of developing PD above 80% independently of CD62E levels. On the other hand, when MIP-1β < 117.06 pg/ml, this critical threshold decreases as the levels of CD62E increase. For the IFN-gamma + MIP-1β model, when MIP-1β > 81.78 pg/ml, patients have a probability of developing PD above 80% independently of IFN-gamma levels. On the other hand, when MIP-1β < 81.78 pg/ml, this critical threshold decreases as the levels of IFN-gamma increase. For the IL-1β + MIP-1β model, when MIP-1β > 91.18 pg/ml, patients have a probability of developing PD above 80% independently of IL-1β levels. On the other hand, when MIP-1β < 91.18 pg/ml, this critical threshold decreases as the levels of IL-1β increase. For the IL-12 + MIP-1β model, when MIP-1β > 91.18 pg/ml, patients have a probability of developing PD above 80% independently of IL-12 levels. On the other hand, when MIP-1β < 91.18 pg/ml, this critical threshold decreases as the levels of IL-12 increase.

Again, we investigated the predictive power of the identified cytokine/chemokine combinations through the ROC curve analysis. Results show that ROC curves of models CD62E + IL-8 and MIP-1β + MCP-1 have an AUC very close to 1 (CD62E + IL-8, AUC = 0.97; MIP-1β + MCP-1, AUC = 1), meaning that both models are outstanding predictors of SD outcome (Figure 3B). The AUC of the combination models, and thus the predictive power, is improved compared to the one of the individual cytokines/chemokines.

Other combinations that are outstanding predictors of SD outcome are: MIP-1β + ICAM-1, MIP-1β + CD62E, MIP-1β + IFN-gamma, MIP-1β + IL-1β and MIP-1β + IL-12. The AUC of these models is 1 (MIP-1β + IFN-gamma, AUC = 1) or very close to 1 (MIP-1β + ICAM-1, AUC = 0.98; MIP-1β + CD62E, AUC = 0.96; MIP-1β + IL-1β, AUC = 0.91; MIP-1β + IL-12, AUC = 0.95) (Figure 3B).

Taken all these results together, it clearly indicates that these cytokines, both as individual markers or in combination with other cytokines, can be used as early predictive biomarkers of response to OMO-103 treatment.

## Claims

1. An *in vitro* method for predicting the clinical response of a subject suffering from cancer to an anti-cancer treatment selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
said method comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value, wherein:
- a decreased level of said at least one biomarker with respect to the reference value is indicative of a good clinical response of the subject to the anti-cancer treatment, or
- an equal or an increased level of said at least one biomarker with respect to the reference value is indicative of a poor clinical response of the subject to the anti-cancer treatment.

2. The method according to claim 1, wherein the good clinical response is stabilization of the disease and the poor clinical response is progression of the disease.

3. An *in vitro* method for selecting a customized therapy for a subject suffering from cancer comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value,
wherein:
- a decreased level of said at least one biomarker with respect to the reference value is indicative that the therapy to be selected comprises an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b); or, alternatively,
- an equal or an increased level of said at least one biomarker with respect to the reference value is indicative that the therapy to be selected does not comprise an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b).

4. An *in vitro* method for selecting a subject suffering from cancer for a treatment comprising an agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
said method comprising:
(i) determining the level of at least one biomarker selected from the group consisting of MIP-1β, CD62E, IL-8, GM-CSF and IL-1α in a sample from said subject; and
(ii) comparing the level of said at least one biomarker with a reference value, wherein the patient is selected for said treatment if a decreased level of said at least one biomarker with respect to the reference value is detected.

5. The method according to any one of claims 1 to 4, wherein the sample is blood, serum or plasma.

6. The method according to any one of claims 1 to 5, wherein the levels of at least one biomarker that are determined are mRNA levels or protein levels.

7. The method according to claim 6, wherein the level of at least one biomarker protein is determined by an immunoassay.

8. The method according to any one of claims 1 to 7, wherein the anti-cancer treatment comprises the use of a polypeptide consisting of SEQ ID NO: 4.

9. The method according to any one of claims 1 to 8, further comprising determining the level of at least one additional biomarker selected from the group consisting of MCP-1, ICAM-1, IFN-gamma, IL-1β, IL-12 and combinations thereof.

10. An agent selected from the group consisting of:
a) a polypeptide comprising the sequence SEQ ID NO: 1 or a functionally equivalent variant thereof;
b) a conjugate comprising a polypeptide comprising the sequence SEQ ID NO: 1 or a functionally equivalent variant thereof and a chemical moiety that facilitates cellular uptake of the polypeptide or of the functionally equivalent variant thereof;
c) a polynucleotide encoding the polypeptide of a) or the conjugate of b);
d) a vector comprising the polynucleotide according to c); and
e) a cell capable of secreting into the medium the polypeptide according to a) or the conjugate according to b);
for use in the treatment of cancer in a subject, wherein the subject has been identified as a good responder to said agent by a method as defined in any one of claims 1 or 2 or 6 to 10 or wherein the treatment has been selected by a method as defined in any one of claims 3 or 5 to 9.

11. . A kit comprising a reagent specific for determining the expression level of at least one biomarker selected from the group consisting of MIP-1β, CD62E, IL-8, GM-CSF and IL-1α.

12. The kit according to claim 11, further comprising reagents specific for determining the expression level of at least one additional biomarker selected from the group consisting of MCP-1, ICAM-1, IFN-gamma, IL-1β, IL-12 and combinations thereof.

13. The kit according to any one of claims 11 or 12, wherein the reagent is an antibody capable of specifically recognizing at least one of the biomarkers.

14. The kit according to any one of claims 11 to 13, wherein the reagent specific for determining the expression level of at least one biomarker comprises at least 10% of the total amount of reagents forming the kit.

15. Use of a kit according to any one of claims 11 to 14 or of a reagent specific for determining the expression level of at least one biomarker selected from the group consisting of MIP-1β, CD62E, IL-8, GM-CSF IL-1α, MCP-1, ICAM-1, IFN-gamma, IL-1β, and IL-12 in the method as defined in any one of claims 1 to 9.
